# EUROPEAN PATENT APPLICATION

(11) **EP 4 696 945 A1**
(43) Date of publication of application: **18.02.2026**
(21) Application number: 25192140.9
(22) Date of filing: 28.07.2025
(51) Int. Cl.: F24F 11/00, F24F 3/167, F24F 110/40, F24F 110/50, F24F 110/66, F24F 110/70

(54) **POSITIVE PRESSURE CONTROL METHOD OF INDOOR AIR CLEANING NETWORK MECHANISM SYSTEM**

(30) Priority: 14.08.2024 TW 113130562
(71) Applicant: Microjet Technology Co., Ltd., Hsinchu (TW)
(72) Inventor: Mou, Hao-Jan, Hsinchu (TW); Wu, Chin-Chuan, Hsinchu (TW); Huang, Chi-Feng, Hsinchu (TW)
(74) Representative: Uexküll & Stolberg

(57) **Abstract**

A positive pressure control method of indoor air cleaning network mechanism system is provided and includes: providing a plurality of gas detectors (1); providing at least one indoor air pollution processing device (2); providing a networked cloud computing service device (3); receiving the carbon dioxide (CO2) pressure detection information and intelligently selecting to issue the control instruction by the networked cloud computing service device (3); comparing whether the pressure difference of the carbon dioxide (CO2) in the indoor field (A) and the outdoor field (B) reach the balance of zero pressure difference; and receiving the control instruction to control the actuation operation of the air guiding fan (21) by the gas detectors (1) inside the indoor air pollution processing device (2), wherein the internal circulation air pollution purification and the temperature and humidity adjustment of the indoor field (A) are implemented continuously, whether the pressure difference of carbon dioxide (CO₂) between the indoor field (A) and the outdoor field (B) is compared to reach the balance of zero pressure difference, the air guiding fan (21) of the indoor air pollution processing device (2) is controlled to adjust the speed reduction of the air guide volume, so that the instant detection of air pollution purification and the complete clean room treatment are realized, and the cleanliness of cleanroom grade is achieved.

## Description

### FIELD OF THE INVENTION

The present disclosure relates to a positive pressure control method of indoor air cleaning network mechanism system, and more particularly to a positive pressure control method of indoor air cleaning network mechanism system for implementing air pollution detection and complete purification in an indoor field.

### BACKGROUND OF THE INVENTION

Suspended particles are defined as the solid particles or droplets contained in the air. Due to their extremely fine size, the suspended particles may enter the lungs of human body through the nasal hair in the nasal cavity easily, causing inflammation in the lungs, asthma or cardiovascular disease. If other pollutant compounds are attached to the suspended particles, it will further increase the harm to the respiratory system. In recent years, the issue of air pollution has been increasingly severe, especially with consistently high concentrations of suspended particles (e.g., PM2.5). Therefore, the monitoring to the concentration of the gas suspended particles is taken more and more seriously. However, the gas flows unstably due to the variable wind direction and the air volume, and the general gas-quality monitoring station is located in a fixed place. Under this circumstance, it is impossible for people to check the concentration of suspended particles in current environment.

Furthermore, in recent years, modern people are placing increasing importance on the quality of the air in their surroundings. For example, carbon monoxide, carbon dioxide, volatile organic compounds

(VOC), PM2.5, nitric oxide, sulfur monoxide and even the suspended particles contained in the air are exposed in the environment to affect the human health, and even endanger the life seriously. Therefore, the quality of environmental air has attracted the attention of various countries. At present, how to detect the air quality and avoid the harm is a crucial issue that urgently needs to be solved.

In order to confirm the quality of the air, it is feasible to use a gas sensor to detect the air surrounding in the environment. If the detection information can be provided in real time to warn the people in the environment, it is helpful of avoiding the harm and facilitates the people to escape the hazard immediately, preventing the hazardous gas exposed in the environment from affecting the human health and causing the harm. Therefore, it is considered a valuable application to use a gas sensor detecting the air in the surrounding environment.

In addition, it is difficult to have the surveillance and control the indoor air quality. Besides the outdoor air quality, the indoor air-conditioning conditions and the pollution sources are the major factors affecting the indoor air quality. It is necessary to intelligently and quickly detect indoor air pollution sources in various indoor fields, effectively remove the indoor air pollution to form a clean and safe breathing gas state, and monitor indoor air quality in real time anytime, anywhere. Certainly, if the concentration of the suspended particles in the space of the indoor field is strictly controlled according to the "Clean Room" standard, it allows to avoid the introduction, generation and retention of suspended particles, and the temperature and humidity in the space of the indoor field are controlled within the required range. That is to say, the number of suspended particles in the air pollution of the space of the indoor field is used to distinguish their classifications, so that it allows the space of the indoor field to meet the clean room requirements for safe breathing.

In view of this, a positive pressure control method of indoor air cleaning network mechanism system is required to detect the indoor air quality in the space of the indoor field and solve the problem of air pollution. Thereby, it allows to achieve real-time detection of air pollution, purification and cleanroom treatment in the indoor field. At the same time, it provides ventilation to prevent the air pollution in the outdoor field from entering the indoor field, so as to achieve the requirements of clean room in the indoor field, and avoid the impact and harm to human health caused by the hazards of gases in the environment.

### SUMMARY OF THE INVENTION

One object of the present disclosure is to provide a positive pressure control method of indoor air cleaning network mechanism system. A plurality of gas detectors are arranged in the indoor field and the outdoor field to detect air pollution information, carbon dioxide (CO₂) pressure detection information and temperature and humidity information of the air. At least one indoor air pollution processing device comprises at least one gas exchange device for implementing ventilation in the indoor field, at least one purification filter device for implementing complete clean room treatment, and at least one air conditioning device for implementing temperature and humidity adjustment in the indoor field. Each indoor air pollution processing device comprises at least one air guiding fan, at least one filter component and at least one driving controller. A networked cloud computing service device is provided to receive the air pollution information of the indoor field and the outdoor field, the carbon dioxide (CO₂) pressure detection information and the temperature and humidity information of the air through Internet of Things communication, and intelligently selecting and issuing a control instruction. Each indoor air pollution processing device with gas detectors is electrically connected to the networked cloud computing service device, so that an intelligent linkage system is formed. Real-time intelligent linkage control between the gas detectors in the indoor field and the outdoor field and the gas detector of the air pollution processing device is implemented to control actuation of the indoor air pollution processing device. In that, the air quality, the temperature and the humidity of space in the indoor felid are monitored anytime and anywhere, and the air pollution is guided to implement the purification and filtration. Meanwhile, the gas exchange device can obtain the result that whether the pressure difference of the carbon dioxide (CO₂) in the indoor field A and the outdoor field B reach the balance of zero pressure difference. Consequently, the positive pressure air intake is continuously provided in the indoor field to implement ventilation in indoor space, so that it prevents the air pollution in the outdoor field from entering the indoor field. When the balance of zero pressure difference is reached, the air guiding fan of the indoor air pollution processing device is controlled in real time to adjust the speed reduction of the air guide volume, the energy-saving efficiency of the operation is effectively adjusted, and the air flow volume noise reaches zero specification value. Consequently, the instant detection of air pollution purification and the complete clean room treatment are realized, and the cleanliness of cleanroom grade is achieved.

In accordance with an aspect of the present disclosure, a positive pressure control method of indoor air cleaning network mechanism system is provided, and includes: a) providing a plurality of gas detectors arranged in an indoor field and an outdoor field to detect air pollution information, carbon dioxide (CO₂) pressure detection information and temperature and humidity information of the air; b) providing at least one indoor air pollution processing device arranged in the indoor field, and having at least one of the plurality of gas detectors disposed therein, wherein the indoor air pollution processing device comprises an air guiding fan, a filter component and a driving controller, and the gas detector is electrically connected to the driving controller, and receives the control instruction to the driving controller through the Internet of Things communication to control actuation operation of the air guiding fan, wherein the indoor air pollution processing device comprises at least one gas exchange device for implementing ventilation in the indoor field, at least one purification filter device for implementing complete clean room treatment, and at least one air conditioning device for implementing temperature and humidity adjustment in the indoor field; c) providing a networked cloud computing service device receiving the air pollution information of the indoor field and the outdoor field, the carbon dioxide (CO₂) pressure detection information and the temperature and humidity information of the air through Internet of Things communication to store and form an air pollution big data database, and intelligently selecting and issuing a control instruction; d) receiving the carbon dioxide (CO₂) pressure detection information and intelligently selecting to issue the control instruction by the networked cloud computing service device, wherein the carbon dioxide (CO₂) pressure detection information detected by the gas detectors disposed inside the gas exchange device, the gas detectors arranged in the indoor field, and the gas detectors arranged in the outdoor field are transmitted to the networked cloud computing service device through the Internet of Things communication, and intelligently calculates and compares the carbon dioxide (CO₂) pressure difference between the indoor field and the outdoor field according to the above carbon dioxide (CO₂) pressure detection information, so as to implement ventilation in indoor space; e) comparing whether the pressure difference of the carbon dioxide (CO₂) in the indoor field and the outdoor field reach the balance of zero pressure difference, wherein if the balance of zero pressure difference is not reached, the control instruction is selectively issued to the gas detector of the gas exchange device, and the gas detector of the gas exchange device receives the control instruction to control the actuation operation of the air guiding fan through the driving controller, the positive pressure air intake is continuously provided in the indoor field to implement ventilation in indoor space, so that it prevents the air pollution in the outdoor field from entering the indoor field, when the balance of zero pressure difference is reached, the control instruction is issued to the gas detector of the gas exchange device, and the gas detector of the gas exchange device receives the control instruction to stop the actuation operation of the air guiding fan through the driving controller; and f) receiving the control instruction and controlling the driving controller to control the actuation operation of the air guiding fan by the gas detectors inside the purification filter device and the air conditioning device, wherein the internal air pollution of the indoor field is continuously circulated and purified, so that the internal circulation air pollution purification and complete clean room treatment and the temperature and humidity adjustment of the indoor field are implemented continuously, when the networked cloud computing service device compares the pressure difference of carbon dioxide (CO₂) between the indoor field and the outdoor field to reach the balance of zero pressure difference, the networked cloud computing service device issues the control instruction to the gas detectors inside the purification filter device and the air conditioning device to control the air guiding fan through the driving controller, wherein the air guiding fan is controlled in real time to adjust the speed reduction of the air guide volume, so that the instant detection of air pollution purification and the complete clean room treatment are realized, and the cleanliness of cleanroom grade is achieved.

### BRIEF DESCRIPTION OF THE DRAWINGS

FIG. 1A is a schematic view illustrating a positive pressure control method of indoor air cleaning network mechanism system in an indoor field according to an embodiment of the present disclosure;
FIG. 1B is a schematic view illustrating a gas exchange device of an indoor air pollution processing device according to the embodiment of the present disclosure;
FIG. 1C is a schematic view illustrating a fan filter unit (FFU) of the indoor air pollution processing device according to the embodiment of the present disclosure;
FIG. 1D is a schematic view illustrating a purification filter device of the indoor air pollution processing device according to the embodiment of the present disclosure;
FIG. 1E is a schematic cross-sectional view illustrating the purification filter device of the indoor air pollution processing device in FIG. 1A and FIG. 1B of the present disclosure;
FIG. 1F is a schematic view illustrating a dehumidifier of the indoor air pollution processing device in FIG. 1A and FIG. 1B according to the embodiment of the present disclosure;
FIG. 1G is a schematic view illustrating a vacuum cleaner of the indoor air pollution processing device in FIG. 1A and FIG. 1B according to the embodiment of the present disclosure;
FIG. 1H is a schematic diagram of the architecture of the process of comparing whether the pressure difference of the carbon dioxide (CO₂) in the indoor field and the outdoor field reach the balance to zero pressure difference and controlling the positive pressure air intake of the present disclosure;
FIG. 2 is a schematic cross-sectional view illustrating a filter component of the indoor air pollution processing device according to the embodiment of the present disclosure;
FIG. 3A is a schematic perspective view illustrating the gas detector according to the embodiment of the present disclosure;
FIG. 3B is a schematic perspective view illustrating the gas detector according to the embodiment of the present disclosure and taken from another perspective;
FIG. 3C is a schematic perspective view illustrating the gas detection module installed inside the gas detector according to the embodiment of the present disclosure;
FIG. 4A is a schematic perspective view (1) illustrating the gas detection main part according to the embodiment of the present disclosure;
FIG. 4B is a schematic perspective view (2) illustrating the gas detection main part according to the embodiment of the present disclosure;
FIG. 4C is an exploded view illustrating the gas detection device according to the embodiment of the present disclosure;
FIG. 5A is a schematic perspective view (1) illustrating the base according to the embodiment of the present disclosure;
FIG. 5B is a schematic perspective view (2) illustrating the base according to the embodiment of the present disclosure;
FIG. 6 is a schematic view (3) illustrating the base according to the embodiment of the present disclosure;
FIG. 7A is a schematic exploded view illustrating the combination of the piezoelectric actuator and the base according to the embodiment of the present disclosure;
FIG. 7B is a schematic perspective view illustrating the combination of the piezoelectric actuator and the base according to the embodiment of the present disclosure;
FIG. 8A is a schematic exploded view (1) illustrating the piezoelectric actuator according to the embodiment of the present disclosure;
FIG. 8B is a schematic exploded view (2) illustrating the piezoelectric actuator according to the embodiment of the present disclosure;
FIG. 9A is a schematic cross-sectional view (1) illustrating an action of the piezoelectric actuator according to the embodiment of the present disclosure;
FIG. 9B is a schematic cross-sectional view (2) illustrating an action of the piezoelectric actuator according to the embodiment of the present disclosure;
FIG. 9C is a schematic cross-sectional view (3) illustrating an action of the piezoelectric actuator according to the embodiment of the present disclosure;
FIG. 10A is a schematic cross-sectional view (1) illustrating the gas detection main part according to the embodiment of the present disclosure;
FIG. 10B is a schematic cross-sectional view (2) illustrating the gas detection main part according to the embodiment of the present disclosure;
FIG. 10C is a schematic cross-sectional view (3) illustrating the gas detection main part according to the embodiment of the present disclosure;
FIG. 11 is a schematic diagram illustrating the communication transmission of the gas detector according to the embodiment of the present disclosure;
FIG. 12A is a schematic diagram of the architecture of a networked cloud computing service device according to the embodiment of the present disclosure;
FIG. 12B is a schematic diagram of the architecture of an artificial intelligence generated content (AIGC) mode according to the embodiment of the present disclosure; and
FIG. 13 is a table showing the required equivalent of the clean air delivery rate (CADR) at the clean room level of ZAPClean Room 1~12 of the present disclosure.

### DETAILED DESCRIPTION OF THE PREFERRED EMBODIMENT

The present disclosure will now be described more specifically with reference to the following embodiments. It is to be noted that the following descriptions of preferred embodiments of this invention are presented herein for purpose of illustration and description only. It is not intended to be exhaustive or to be limited to the precise form disclosed.

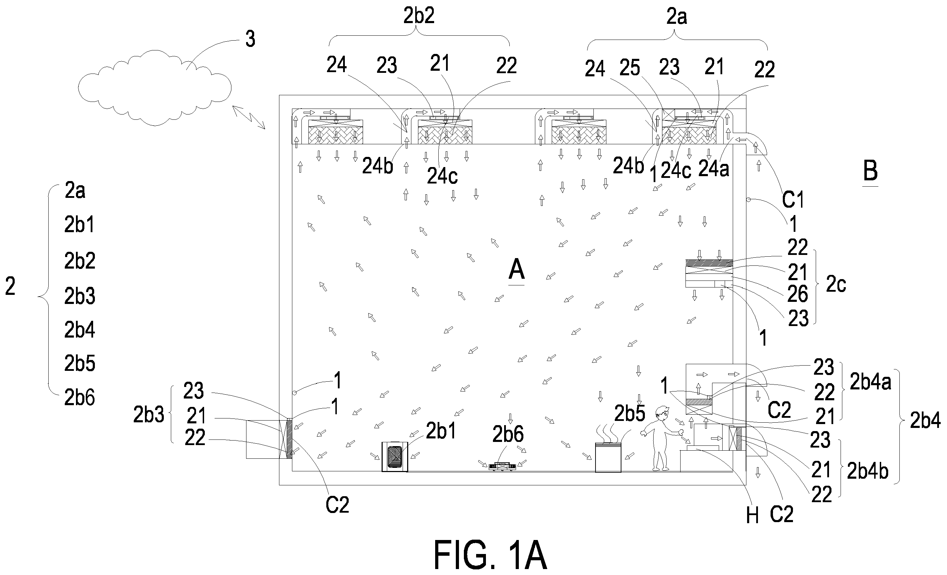

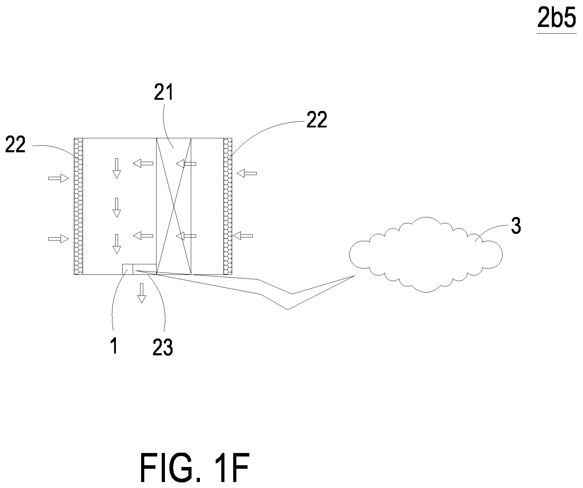

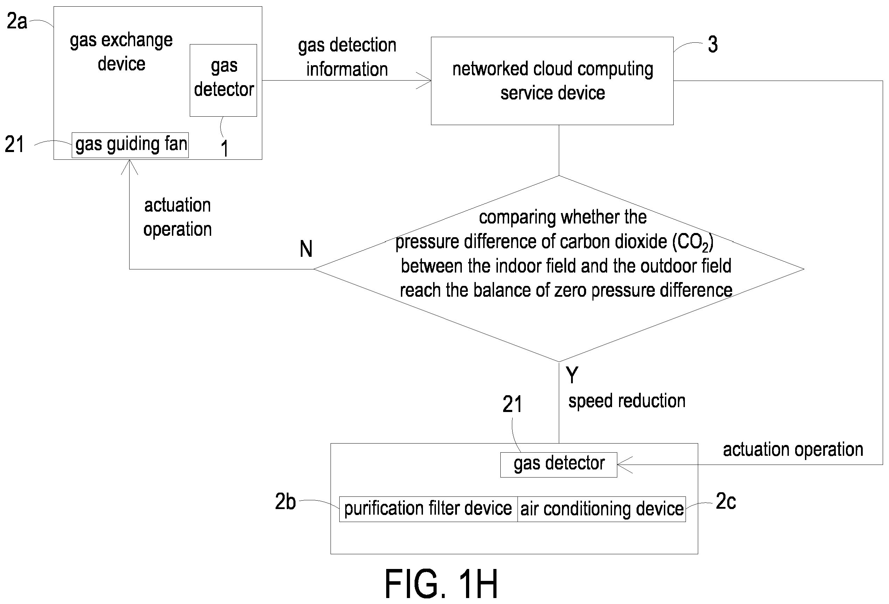

The present disclosure provides a positive pressure control method of indoor air cleaning network mechanism system, which comprises:
a) Providing a plurality of gas detectors 1 arranged in an indoor field A and an outdoor field B to detect air pollution information, carbon dioxide (CO₂) pressure detection information and temperature and humidity information of the air. As shown in FIG. 1A, the plurality of gas detectors 1 are arranged in the indoor field A and the outdoor field B to detect the air pollution information, the carbon dioxide (CO₂) pressure detection information and the temperature and humidity information of the air, and the gas detector transmits the air pollution information, the carbon dioxide (CO₂) pressure detection information and the temperature and humidity information of the air through Internet of Things (IoT) communication.
b) Providing at least one indoor air pollution processing device 2 arranged in the indoor field A, and having at least one of the plurality of gas detectors 1 disposed therein. The indoor air pollution processing device 2 comprises at least one air guiding fan 21, at least one filter component 22 and at least one driving controller 23. The gas detector 1 is electrically connected to the driving controller 23, and receives the control instruction through the IoT communication to control actuation operation of the air guiding fan 21. The indoor air pollution processing device 2 comprises at least one gas exchange device 2a for implementing ventilation in the indoor field, at least one purification filter device 2b for implementing complete clean room treatment, and at least one air conditioning device 2c for implementing temperature and humidity adjustment in the indoor field A.
c) Providing a networked cloud computing service device 3. As shown in FIG. 1H. The networked cloud computing service device 3 receives the air pollution information of the indoor field A and the outdoor field B, the carbon dioxide (CO₂) pressure detection information and the temperature and humidity information of the air through the IoT communication to store and form an air pollution big data database, and intelligently selecting and issuing a control instruction.
d) Receiving the carbon dioxide (CO₂) pressure detection information and intelligently selecting to issue the control instruction by the networked cloud computing service device 3. The carbon dioxide (CO₂) pressure detection information detected by the gas detectors 1 disposed inside the gas exchange device 2a, the gas detectors 1 arranged in the indoor field A, and the gas detectors 1 arranged in the outdoor field B are transmitted to the networked cloud computing service device 3 through the IoT communication. The networked cloud computing service device 3 intelligently calculates and compares the carbon dioxide (CO₂) pressure difference between the indoor field A and the outdoor field B according to the above carbon dioxide (CO₂) pressure detection information, so as to implement ventilation in indoor space A.
e) Comparing whether the pressure difference of the carbon dioxide (CO₂) in the indoor field A and the outdoor field B reach the balance of zero pressure difference. If the balance of zero pressure difference is not reached, the control instruction is selectively issued to the gas detector 1 of the gas exchange device 2a. The gas detector 1 of the gas exchange device 2a receives the control instruction to control the actuation operation of the air guiding fan 21 through the driving controller 23. The positive pressure air intake is continuously provided in the indoor field A to implement ventilation in indoor space, so that it prevents the air pollution in the outdoor field B from entering the indoor field A. When the balance of zero pressure difference is reached, the control instruction is issued to the gas detector 1 of the gas exchange device 2a. The gas detector 1 of the gas exchange device 2a receives the control instruction to stop the actuation operation of the air guiding fan 21 through the driving controller 23.
f) Receiving the control instruction and controlling the driving controller 23 to control the actuation operation of the air guiding fan 21 by the gas detectors 1 inside the purification filter device 2b and the air conditioning device 2c. The internal air pollution of the indoor field A is continuously circulated and purified, so that the internal circulation air pollution purification and complete clean room treatment and the temperature and humidity adjustment of the indoor field A are implemented continuously. When the networked cloud computing service device 3 compares the pressure difference of carbon dioxide (CO₂) between the indoor field A and the outdoor field B to reach the balance of zero pressure difference, the networked cloud computing service device 3 issues the control instruction to the gas detectors 1 inside the purification filter device 2b and the air conditioning device 2c to control the air guiding fan 21 through the driving controller 23. In that, the air guiding fan 21 is controlled in real time to adjust the speed reduction of the air guide volume, so that the instant detection of air pollution purification and the complete clean room treatment are realized. Meanwhile, the cleanliness of cleanroom grade is achieved.

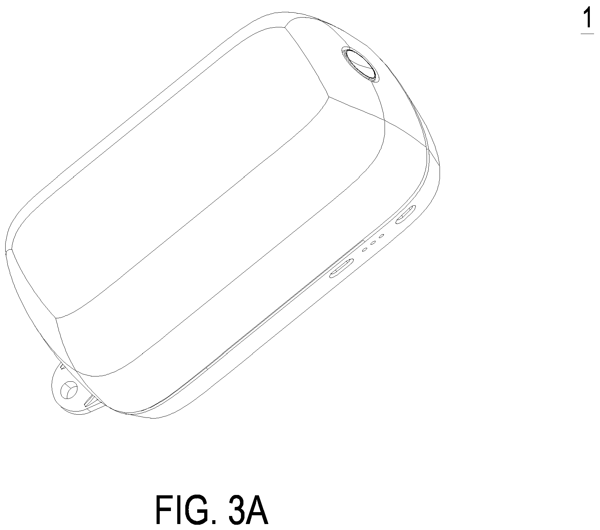

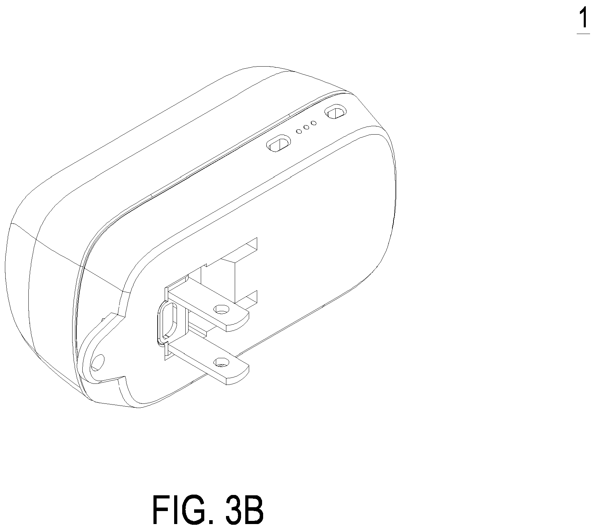

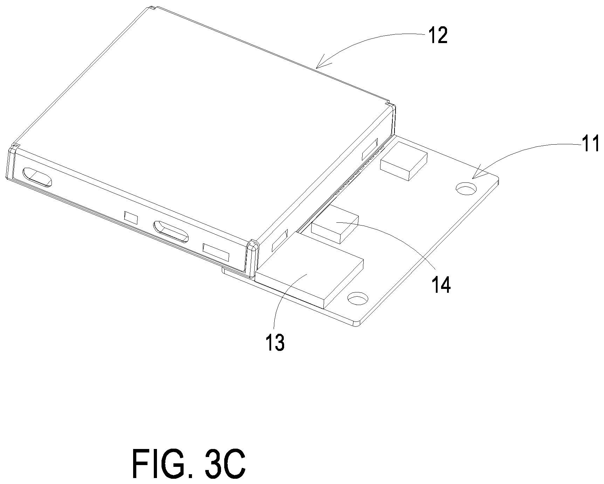

Notably, in the embodiment, the gas detector 1 includes a gas detection module disposed therein. Please refer to FIG. 3A and FIG. 3B. Preferably but not exclusively, the gas detector 1 is formed by a type of structure including an external power terminal, which can be directly inserted into the power interface in the indoor field A to start the operation of detecting the air pollution information, the carbon dioxide (CO₂) pressure detection information, and the gas temperature and humidity information. In an embodiment, as shown in FIG. 3C, a type of the gas detection module without an external power terminal is directly constructed on and electrically connected to an indoor air pollution processing device 2. A control instruction is received to control the power supply and the actuation operation of the indoor air pollution processing device 2. Notably, in the embodiment, the air pollution gas is at least one selected from the group consisting of particulate matter, carbon monoxide, carbon dioxide, ozone, sulfur dioxide, nitrogen dioxide, lead, total volatile organic compounds (TVOC), formaldehyde, bacteria, fungi, virus and a combination thereof.

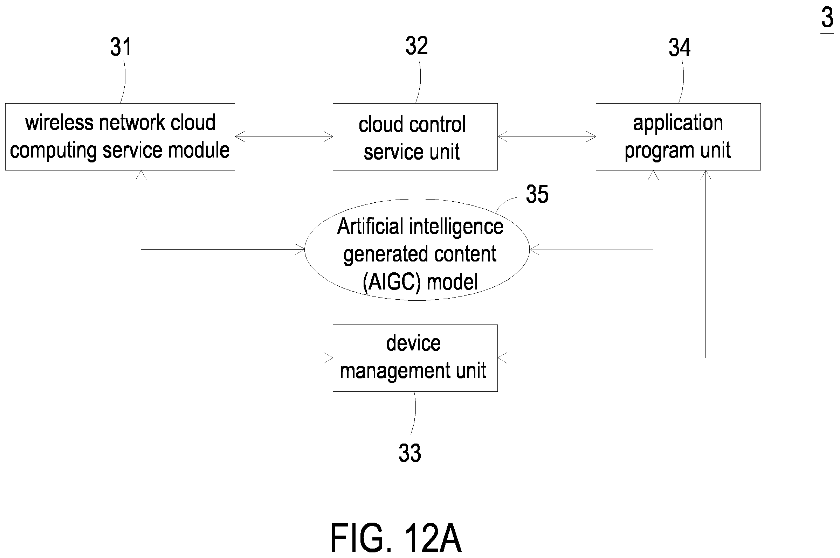

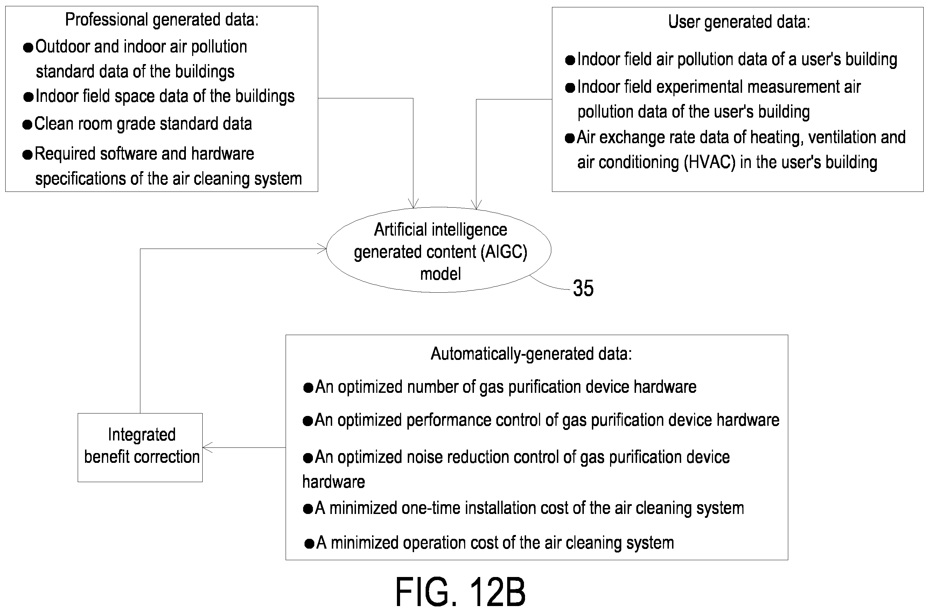

Notably, in the embodiment, the Internet of Things communication refers to a collective network, which connects various devices and technologies and helps the devices communicate with the cloud and with each other. Preferably but not exclusively, the IoT communication is a wired communication, which is connected to the networked cloud computing service device 3 via a wired line. Preferably but not exclusively, the IoT communication is a wireless communication for communicating with the networked cloud computing service device 3 via a wireless connection. The wireless communication transmission includes one selected from the group consisting of a Wi-Fi module, a Bluetooth module, a radio frequency identification module, and a near field communication (NFC) module. As shown in FIG. 12A and FIG. 12B, the networked cloud computing service device 3 includes a wireless network cloud computing service module 31, a cloud control service unit 32, a device management unit 33, an application unit 34 and an artificial intelligence generated content (AIGC) model 35. Preferably but not exclusively, the wireless network cloud computing service module 31 receives the air pollution information, the carbon dioxide (CO₂) pressure detection information and the gas temperature and humidity information of the indoor field A and the outdoor field B, receives the communication information of the devices as shown in FIG. 1A, and transmits the control instructions. Moreover, the wireless network cloud computing service module 31 receives the air pollution information, the carbon dioxide (CO₂) pressure detection information and the gas temperature and humidity information of the indoor field A and the outdoor field B and transmits it to the cloud control service unit 32 to store and form an air pollution big data database. An artificial intelligence calculation is implemented to determine the location of the air pollution through the air pollution database comparison, so that the control commend is transmitted to the wireless network cloud computing service module 31, and then transmitted to the indoor air pollution processing device 2 to control the actuation operation through the wireless network cloud computing service module 31. The device management unit 33 receives the communication information of the devices through the wireless network cloud computing service module 31 to manage the user login and device binding. The device management information can be provided to the application program unit 34 for system control and management, and the application program unit 34 can also display and inform the air pollution information, the carbon dioxide (CO₂) pressure detection information, and the gas temperature and humidity information obtained by the cloud control service unit 32. The user can know the real-time status of air pollution removal through the mobile phone or the communication device. Moreover, the user can control the operation of the indoor air cleaning system through the application program unit 34 of the mobile phone or the communication device. In the embodiment, the artificial intelligence generated content (AIGC) mode 35 provides professional generated data to the indoor air cleaning network mechanism system. Preferably but not exclusively, in the embodiment, the professional generated data includes outdoor and indoor air pollution standard data of the buildings, indoor field space data of the buildings, clean room grade standard data, and required software and hardware specifications. In the embodiment, the artificial intelligence generated content (AIGC) mode 35 provides user generated data to the indoor air cleaning network mechanism system. Preferably but not exclusively, in the embodiment, the user generated data includes indoor field air pollution data of a user's building, indoor field experimental measurement air pollution data of the user's building, and air exchange rate data of heating, ventilation and air conditioning (HVAC) in the user's building. In the embodiment, the artificial intelligence generated content (AIGC) mode 35 calculates, compares and identifies the professional generated data and the user generated data to form automatically-generated data. Preferably but not exclusively, the automatically-generated data includes the optimized number of gas purification device hardware, the optimized performance control of gas purification device hardware, the optimized noise reduction control of gas purification device hardware, the minimized one-time installation cost of the air cleaning system and the minimized operation cost of the air cleaning system.

Notably, as shown in FIG. 1A. The present disclosure provides at least one indoor air pollution processing device 2 arranged in the indoor field A. Each indoor air pollution processing device 2 includes at least one gas detector 1, at least one air guiding fan 21, at least one filter component 22 and at least one driving controller 23 disposed therein disposed inside. The driving controller 23 and the gas detector 1 are electrically connected to each other. The gas detector 1 receives a control instruction from the networked cloud computing service device 3 through Internet of Things (IoT) communication to control actuation operation of the air guiding fan 21 through the driving controller 23. In the embodiment, the indoor air pollution processing device 2 comprises at least one gas exchange device 2a for implementing ventilation in the indoor field, at least one purification filter device 2b for implementing complete clean room treatment, and at least one air conditioning device 2c for implementing temperature and humidity adjustment in the indoor field A. Preferably but not exclusively, the purification filter device 2b includes at least one purifier 2b1, at least one fan filter unit (FFU) 2b2, at least one exhaust device 2b3, at least one smoke exhaust system 2b4, at least one dehumidifier 2b5, and at least one mobile vacuum cleaner 2b6. In the embodiment, the gas detector 1 arranged inside the indoor air pollution processing device 2 is electrically connected to the driving controller 23, and receives the control instruction to the driving controller 23 through the Internet of Things communication to control actuation operation of the air guiding fan 21.

Notably, the networked cloud computing service device 3 receives the carbon dioxide (CO₂) pressure detection information detected by the gas detectors 1 disposed inside the gas exchange device 2a, the gas detectors 1 arranged in the indoor field A, and the gas detectors 1 arranged in the outdoor field B through the IoT communication. The networked cloud computing service device 3 intelligently calculates and compares the carbon dioxide (CO₂) pressure difference between the indoor field A and the outdoor field B according to the above carbon dioxide (CO₂) pressure detection information, so as to implement ventilation in indoor space A. As shown in FIG. 1H. The networked cloud computing service device 3 receives the detection information of the gas exchange device 2a through the IoT communication to compare whether the pressure difference of carbon dioxide (CO₂) in the indoor field A and the outdoor field B reaches the balance of zero pressure difference. If the balance of zero pressure difference is not reached, the control instruction is selectively issued to the gas detector 1 of the gas exchange device 2a, and the gas detector 1 of the gas exchange device 2a receives the control instruction to control the actuation operation of the air guiding fan 21 through the driving controller 23. The positive pressure air intake is continuously provided in the indoor field A to implement ventilation in indoor space A, so that it prevents the air pollution in the outdoor field B from entering the indoor field A. When the balance of zero pressure difference is reached, the control instruction is issued to the gas detector 1 of the gas exchange device 2a, and the gas detector 1 of the gas exchange device 2a receives the control instruction to stop the actuation operation of the air guiding fan 21 through the driving controller 23. In this positive pressure control method of indoor air cleaning network mechanism system, when the gas exchange device 2a is enabled for ventilation, a positive pressure greater than 0 Pa is maintained in the space of the indoor field A to prevent the air pollution in the outdoor field B from entering the indoor field A. In an embodiment, each gas detector 1 inside the purification filter device 2b and the air conditioning device 2c continuously receives the control instruction from the networked cloud computing service device 3 and controls the driving controller 23 to control the actuation operation of the air guiding fan 21, and the internal air pollution of the indoor field A is continuously circulated and purified, so that the internal circulation air pollution purification and complete clean room treatment and the temperature and humidity adjustment of the indoor field A are implemented continuously. When the networked cloud computing service device 3 compares the pressure difference of carbon dioxide (CO₂) between the indoor field A and the outdoor field B to reach the balance of zero pressure difference, the networked cloud computing service device 3 also issues a control instruction to the internal gas detector 1 of the purification filter device 2b and the air conditioning device 2c, and the control instruction is received to control the air guiding fan 21 through the driving controller 23. In that, the air guiding fan 21 is controlled in real time to adjust the speed reduction of the air guide volume according to the air quality, the energy-saving efficiency of the operation is effectively adjusted, and the air flow volume noise reaches zero specification value. Consequently, the instant detection of air pollution purification and the complete clean room treatment are realized, and the cleanliness of cleanroom grade is achieved.

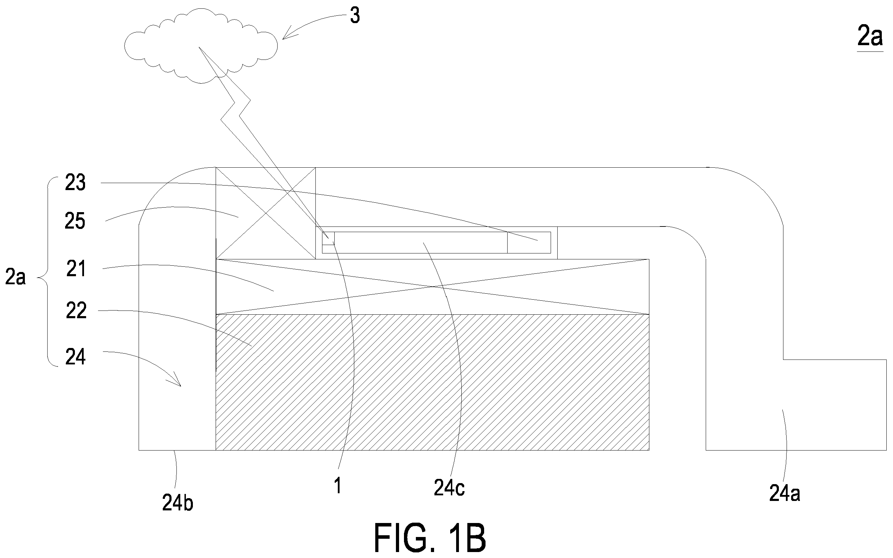

From the above, in the present disclosure, the specific implementation of the positive pressure control method of indoor air cleaning network mechanism system is understandable, and the operation of each indoor air pollution processing device 2 for implementing air pollution detection and complete purification in the space of an indoor field is described below. As shown in FIG. 1A and FIG. 1B. The indoor field A includes at least one air inlet C1 and at least one exhaust port C2. The gas exchange device 2a includes a guide channel 24. The guide channel 24 has an air inlet 24a corresponding to the air inlet C1 of the indoor field A, a circulating return port 24b in communication with the indoor field A, and a filtering air duct 24c in communication with the indoor field A. A gas exchange fan 25 is disposed in the circulating return port 24b, and an air guiding fan 21 and a filter component 22 are disposed in the filtering air duct 24c. In the embodiment, the networked cloud computing service device 3 intelligently calculates and compares the air pollution information, the carbon dioxide (CO₂) pressure detection information and the gas temperature and humidity information of the indoor field A and the outdoor field B. Preferably but not exclusively, when the air pollution information in the indoor field A is higher than that of outdoor space B, the gas detector 1 inside the gas exchange device 2a receives the control instruction from the networked cloud computing service device 3 through the Internet of Things communication, and controls the driving controller 23 to control the actuation operation of the air guiding fan 21 and the gas exchange fan 25, so that the air of the outdoor field B is introduced into the filtering air duct 24c through the air inlet C1, and filtered into the indoor field A through the filter component 22. At the same time, the air of the indoor field A enters the filtering air duct 24c through the circulating return port 24b, and then is circulated and filtered to adjust the temperature and implement the ventilation. The ventilation is implemented to achieve zero pressure balance of the carbon dioxide (CO₂) pressure detection between the indoor field A and the outdoor field B. Notably, when the gas exchange device 2a enable the actuation operation for ventilation, a positive pressure greater than 0 Pa is maintained in the space of the indoor field A to prevent the air pollution in the outdoor field B from entering the indoor field A. In an embodiment, the gas exchange device 2a is a fresh air fan, or the gas exchange device 2a is a full heat exchanger, or the gas exchange device 2a is a heating ventilation and air conditioning (HVAC). The present disclosure is not limited thereto.

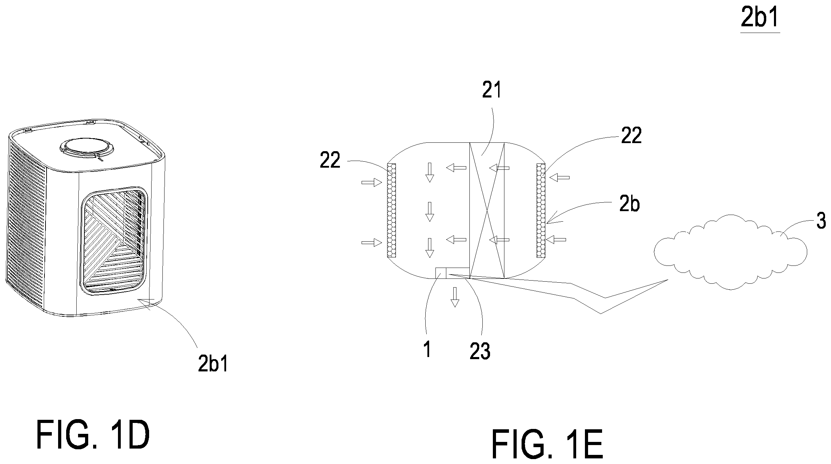

Please refer to FIG. 1A, FIG. 1D and FIG. 1E. In the embodiment, the purifier 2b1 is plugged-in to the space of the indoor field A, and the networked cloud computing service device 3 issues the control instruction to the gas detector 1 inside the purifier 2b1 through the Internet of Things communication. The control instruction is received by the driving controller 23 to control the actuation operation of the air guiding fan 21, so as to guide the air pollution in the space of the indoor field A to be filtered and purified by the filter component 22, and the air purified is then introduced into the space of the indoor field A. Whereby, the air pollution in the space of the indoor field A is guided to pass through the filter component 22 multiple times for air pollution purification and complete clean room treatment.

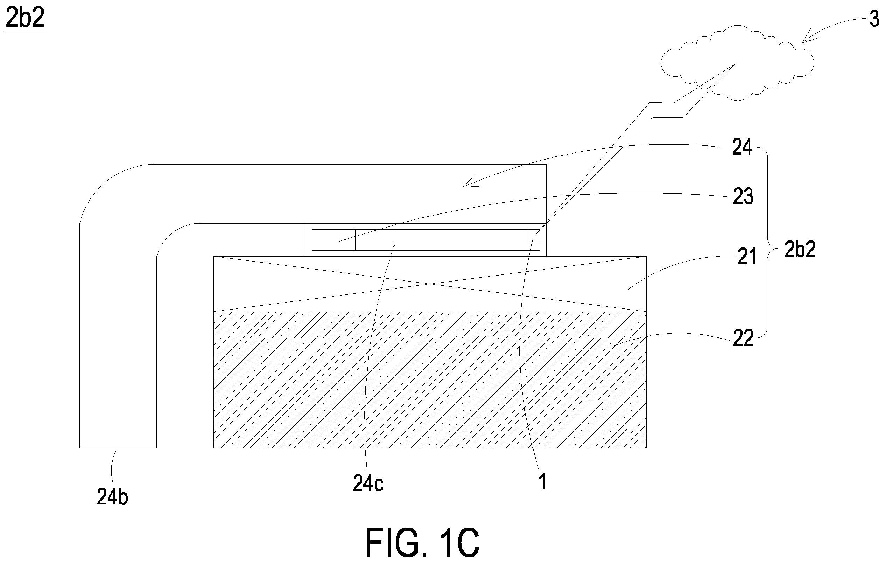

Please refer to FIG. 1A and FIG. 1C. In the embodiment, the fan filter unit (FFU) 2b2 is built-in to the space of the indoor field A. The fan filter unit (FFU) 2b2 includes a guide channel 24. The guide channel 24 has a circulating return port 24b in communication with the indoor field A, and a filtering air duct 24c in communication with the indoor field A. An air guiding fan 21 and a filter component 22 are disposed in the filtering air duct 24c. The networked cloud computing service device 3 issues the control instruction to the gas detector 1 inside the fan filter unit (FFU) 2b2 through the Internet of Things communication. The control instruction is received by the gas detector 1 and transmitted to the driving controller 23 to control the actuation operation of the air guiding fan 21. In that, the air pollution in the space of the indoor field A is guided from the circulating return port 24b into the filtering air duct 24c of the guide channel 24 to be filtered and purified by the filter component 22 inside the fan filter unit (FFU) 2b2. The air purified is then introduced into the space of the indoor field A from the guide channel 24, so that the air pollution in the space of the indoor field A is guided to enter the guide channel 24 multiple times for air pollution purification and complete clean room treatment.

Please refer to FIG. 1A. In the embodiment, the exhaust device 2b3 is built-in to the indoor field A, corresponds to the exhaust port C2 and is in communication with the outdoor field B. The networked cloud computing service device 3 issues the control instruction to the gas detector 1 inside the exhaust device 2b3 through the Internet of Things communication. The control instruction is received by the driving controller 23 to control the actuation operation of the air guiding fan 21 to guide the air pollution in the space of the indoor field A to be introduced by the air guiding fan 21 through the filter component 22 for filtration and purification and discharged to the outdoor field B. Whereby, the air pollution in the space of the indoor field A is purified to achieve the complete clean room treatment.

Moreover, please refer to FIG. 1A. When cooking food in the kitchen environment of indoor field A, serious air pollution is generated relatively quickly. In order to prevent the air pollution generated in indoor field A from affecting human health and causing harm, the purification filter device is set and served as a smoke exhaust system 2b4, which is arranged in the kitchen of indoor field A. The smoke exhaust system 2b4 includes an exhaust channel 2b4a, which corresponds to the exhaust port C2 in communicate with the outdoor field B, and is arranged above a cooking appliance H. The exhaust channel 2b4a is provided with an air guiding fan 21, a filter component 22 and a driving controller 23, and the gas detector 1 is arranged in the exhaust channel 2b4a and electrically connected to the driving controller 23. The gas detector 1 receives the control instruction to the driving controller 23 through the Internet of Things communication to control the actuation operation of the air guiding fan 21. Moreover, the smoke exhaust system 2b4 includes an oil fume exhaust body 2b4b, which corresponds to the exhaust port C2 in communication with the outdoor field B and is arranged in front of the cooking appliance H. The oil fume exhaust body 2b4b is provided with an air guiding fan 21, a filter component 22 and a driving controller 23, and a gas detector 1 is arranged in the oil fume exhaust body 2Ib and electrically connected to the driving controller 23. The control instruction is received by the gas detector 1 to the driving controller 23 through the Internet of Things communication to control the actuation operation of the air guiding fan 21. The networked cloud computing service device 3 issues the control instruction to the gas detector 1 inside the exhaust channel 2b4a and the gas detector 1 inside the oil fume exhaust body 2b4b through the Internet of Things communication. The control instruction is transmitted to the driving controller 23 to control the actuation operation of the air guiding fan 21. The air pollution in the kitchen of the indoor field A flows into the exhaust channel 2b4a and the oil fume exhaust body 2b4b and passes through the filter component 22 therein for filtering and purification, and then introduced into the outdoor field B, so that the kitchen for air pollution purification and complete clean room treatment is achieved.

Please refer to FIG. 1A. In the embodiment, the dehumidifier 2b5 is plugged-in to the space of the indoor field A, and the networked cloud computing service device 3 issues the control instruction to the gas detector 1 inside the dehumidifier 2b5 through the Internet of Things communication. The control instruction is received by the driving controller 23 to control the actuation operation of the air guiding fan 21. The air pollution in the space of the indoor field A is guided by the air guiding fan 21 and purified by the filter component 22 for air pollution purification and complete clean room treatment. Moreover, the temperature and humidity of the air in the indoor field A is adjusted. Notably, the dehumidifier 2b5 sets the temperature and humidity at safety values to maintain the temperature at 25°C±3°C and the humidity at 50%±10%.

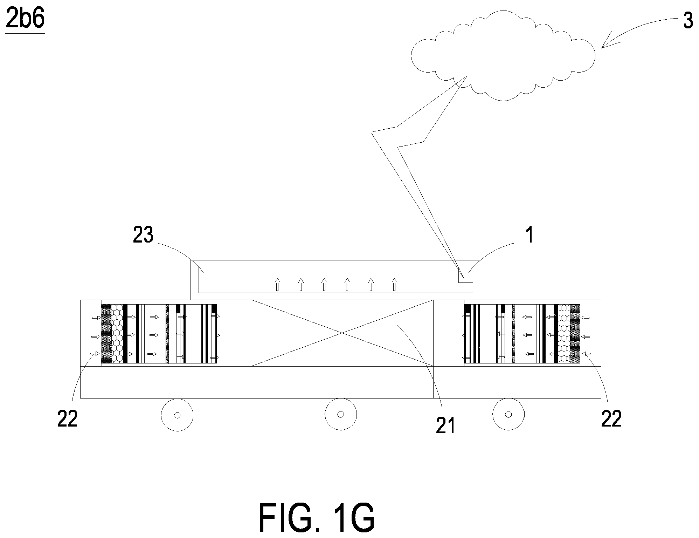

Pease refer to FIG. 1A and FIG. 1G. In the embodiment, the mobile vacuum cleaner 2b6 is plugged-in to the space of the indoor field A, and the networked cloud computing service device 3 issues the control instruction to the gas detector 1 inside the mobile vacuum cleaner 2b6 through the Internet of Things communication. The control instruction is received by the driving controller 23 to control the actuation operation of the air guiding fan 21. The air pollution in the space of the indoor field A is guided to the filter component 22 for air pollution purification and complete clean room treatment. Notably, the mobile vacuum cleaner 2b6 is a sweeping robot.

Pease refer to FIG. 1A. The air conditioning device 2c is arranged in the indoor field A and has at least one gas detector 1 disposed therein. The air conditioning device 2c includes at least one cooling/heat exchanger 26. The gas detector 1 is arranged inside the air conditioning device 2c and electrically connected to the driving controller 23. The gas detector 1 receives the control instruction to the driving controller 23 through the Internet of Things communication to control actuation operation of the air guiding fan 21 of the air conditioning device 2c, so that the air in the indoor field A is guided through the cooling/heat exchanger 26 to adjust a temperature and humidity of the air in the indoor field A, and the gas detector 1 externally transmits the temperature and humidity information of the air in the indoor field A. Notably, the temperature and humidity is set at safety values, that is, the temperature and humidity of the indoor field A is maintained at the temperature of 25°C±3°C and the humidity of 50%±10%.

From the above, the indoor air cleaning network mechanism system of the present disclosure provides the indoor field A with circulating air pollution purification and completely clean room treatment, and achieves the cleanliness of the clean room level. Moreover, a required equivalent of a clean air delivery rate (CADR) in the space of the indoor field A is determined by the artificial intelligence generated content (AIGC) model 35 of the networked cloud computing service device 3. After the intelligent (AI) calculation of the artificial intelligence generated content (AIGC) model 35, the number of equipment matching arrangements and the optimal clean air delivery rate (CADR) of the air guiding fan 21 can be determined according to the required equivalent of the clean air delivery rate (CADR). Consequently, the instant detection of air pollution purification and the complete clean room treatment are realized, the cleanliness of cleanroom grade is achieved, and the cost effectiveness of clean room treatment towards complete purification is optimized.

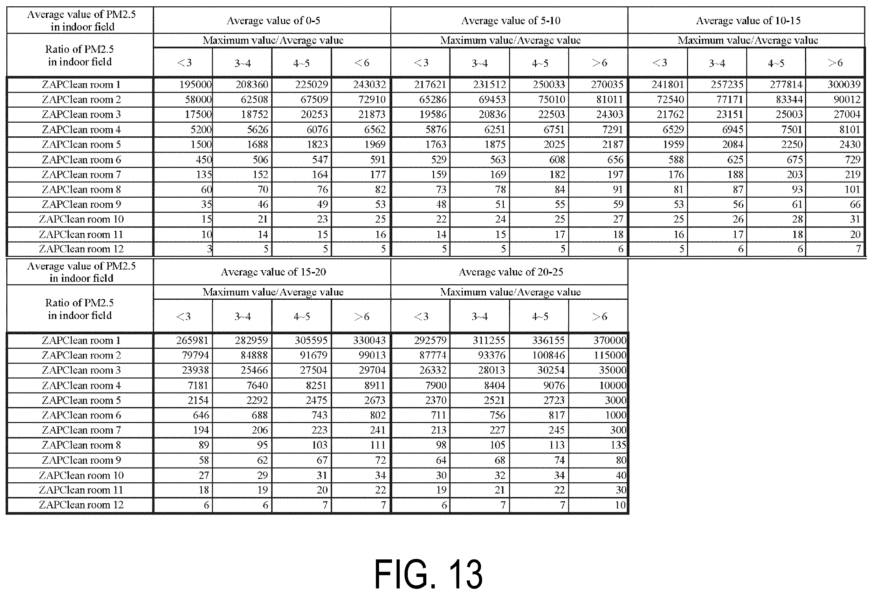

As shown in FIG. 13, the required cleanliness of the indoor field A of the present disclosure needs to meet a clean room level of ZAPClean Room 1~12. Therefore, after the indoor air cleaning network mechanism system uses the intelligent (AI) calculation of the artificial intelligence generated content (AIGC) model 35 to determine the required equivalent of the clean air delivery rate (CADR) for the space of the indoor field A, the number of matching arrangements and the optimal clean air delivery (CADR) of the air guiding fan 21 can be determined according to the required equivalent of the clean air delivery rate (CADR), and the air quality in the space of the indoor field A can be monitored anytime and anywhere. At the same time, the air pollution in the space of the indoor field is detected and intelligently compared with the ambient air quality status, and the air guiding fan 21 is controlled in real time to adjust the speed reduction of the air guide volume according to the air quality, so as to effectively control the energy-saving benefits of the operation of the purification device, achieve the cleanliness of the clean room level and optimize the cost setting benefits of the purification and complete clean room treatment. Notably, the required equivalent of clean air delivery rate (CADR) refers to the clean air delivery rate (CADR) of the air guiding fan 21 required in the space of the indoor field A in this area at that time, which can achieve the demand of completely cleaning the air pollution.

The following is an example of a preferred embodiment of the clean air delivery rate (CADR) required in the space of the indoor space A of the present disclosure:

In the positive pressure control method of indoor air cleaning network mechanism system, as long as the area location of the indoor space is inputted, the required clean air delivery volume (CADR) in the space of the indoor space A can be obtained. If the area location of the indoor space is in Taipei, what is the required clean air delivery volume (CADR) required for the cleanliness level of ZAPClean Room 9 in the 3 square meters of the indoor space?

The big data database of the air pollution prevention system can be used for intelligent calculation and analysis according to the equivalent comparison table of the required clean air delivery rate (CADR) per cubic meter as the clean room levels of ZAPClean Room1~12 in FIG. 13.

The required equivalent of the clean air delivery rate (CADR) per cubic meter for the clean room levels of ZAPClean Room 1~12 of the present disclosure is as follows:
The required equivalent of the clean air delivery rate (CADR) per cubic meter at the clean room level of ZAPClean Room 1 is ranged from 195000 m³/h to 370000 m³/h, the required equivalent of the clean air delivery rate (CADR) per cubic meter at the clean room level of ZAPClean Room 2 is ranged from 58000 m³/h to115000 m³/h, the required equivalent of the clean air delivery rate (CADR) per cubic meter at the clean room level of ZAPClean Room 3 is ranged from 17500 m³/h 35000 m³/h, the required equivalent of the clean air delivery rate (CADR) per cubic meter at the clean room level of ZAPClean Room 4 is ranged from 5200 m³/h 10000 m³/h, the required equivalent of the clean air delivery rate (CADR) per cubic meter at the clean room level of ZAPClean Room 5 is ranged from 1500 m³/h to 3000 m³/h, the required equivalent of the clean air delivery rate (CADR) per cubic meter at the clean room level of ZAPClean Room 6 is ranged from 450 m³/h to 1000 m³/h, the required equivalent of the clean air delivery rate (CADR) per cubic meter at the clean room level of ZAPClean Room 7 is ranged from 135 m³/h to 300 m³/h, the required equivalent of the clean air delivery rate (CADR) per cubic meter at the clean room level of ZAPClean Room 8 is ranged from 60 m³/h to 135 m³/h, the required equivalent of the clean air delivery rate (CADR) per cubic meter at the clean room level of ZAPClean Room 9 is ranged from 35 m³/h to 80 m³/h, the required equivalent of the clean air delivery rate (CADR) per cubic meter at the clean room level of ZAPClean Room 10 is ranged from 15 m³/h to 40 m³/h, the required equivalent of the clean air delivery rate (CADR) per cubic meter at the clean room level of ZAPClean Room 11 is ranged from 10 m³/h to 30 m³/h, and the required equivalent of the clean air delivery rate (CADR) per cubic meter at the clean room level of ZAPClean Room 12 is ranged from 3 m³/h to 10 m³/h.

When the area location of the indoor field A in Taipei and the required space volume are inputted, the artificial intelligence generated content (AIGC) model 35 of the networked cloud computing service device 3 can intelligently calculate and determine the required clean air delivery rate (CADR) for performing air pollution purification and complete clean room treatment. It is calculated that the maximum value of PM2.5 in Taipei over the past five years is 37, and the average value is 11.9. At this time, the average value of 11.9 falls in the average value comparison table of average zone 10~15, and the maximum value is 37/average value 11.9, which is equal to 3.1, which falls in the ratio zone 3~4 of the average zone 10~15 in comparison table. Furthermore, the required cleanliness is at the level of ZAPClean Room 9. Therefore, the required equivalent of the clean air delivery per cubic meter (CADR) for the cleanliness level of ZAPClean Room 9 in this indoor field is 56.26 m³/h. In case of that the required space of the indoor field is 30 ping (268 m³), it is multiplied by 56.26 m³/h, so the required clean air delivery rate (CADR) for this required indoor field is 15078 m³/h. Therefore, the required equivalent of the clean air delivery rate (CADR) of the indoor air pollution processing device 2 for performing air pollution purification and complete clean room treatment is 15000 m³/h. In an embodiment, the indoor air pollution processing device 2 of the present disclosure is configured to combine the three gas exchange devices 2a with the optimal clean air delivery rate (CADR) set at 1000 m³/h, and fifteen air guiding fans 21 of the fan filter units (FFU) 2b2 with the optimal clean air delivery rate (CADR) at 800 m³/h, so as to achieve the required equivalent of clean air delivery rate (CADR) of the indoor air pollution processing device 2 at 15000 m³/h for performing air pollution purification and complete clean room treatment, but not limited to thereto. Certainly, the required equivalent of the clean air delivery rate (CADR) for the indoor field A can be utilized to determine the number of matching arrangements of the indoor air pollution processing device 2 and the optimal clean air delivery rate (CADR) of the air guiding fan 21 of the indoor air pollution processing device 2, so as to realize real-time detection of air pollution purification and complete clean room processing, achieve the cleanliness of the clean room level and optimize the cost-effectiveness of purification and complete clean room treatment.

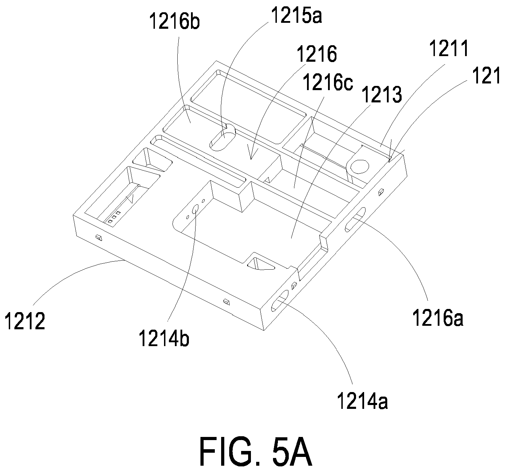

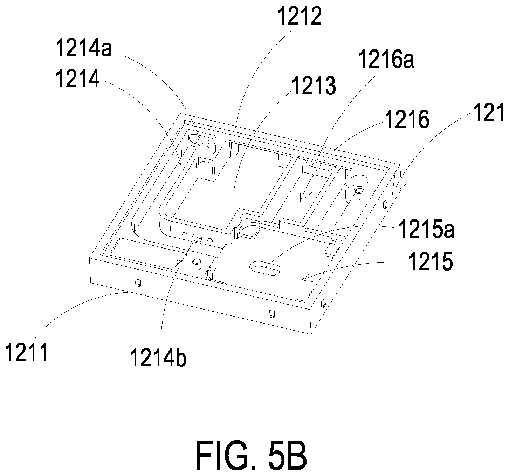

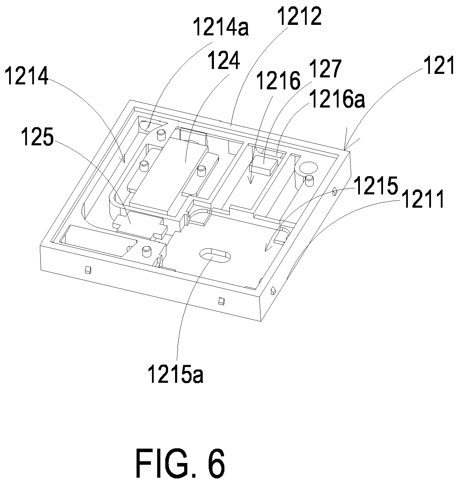

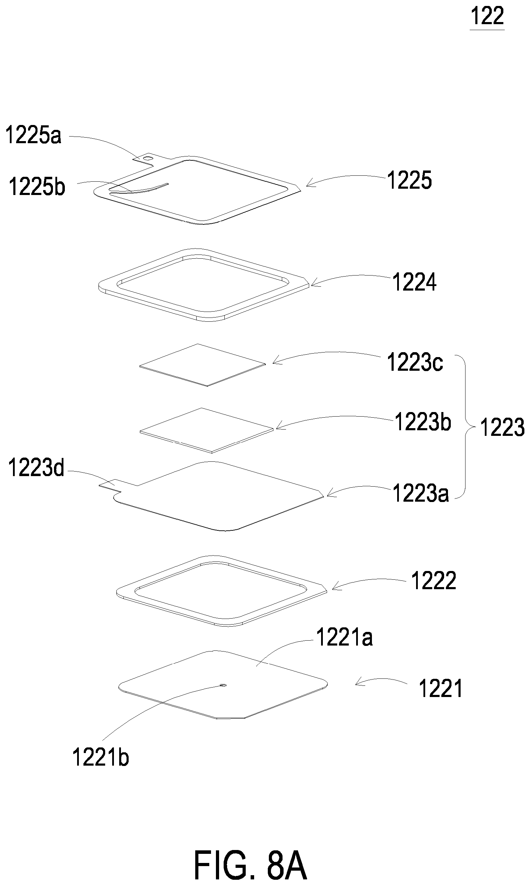

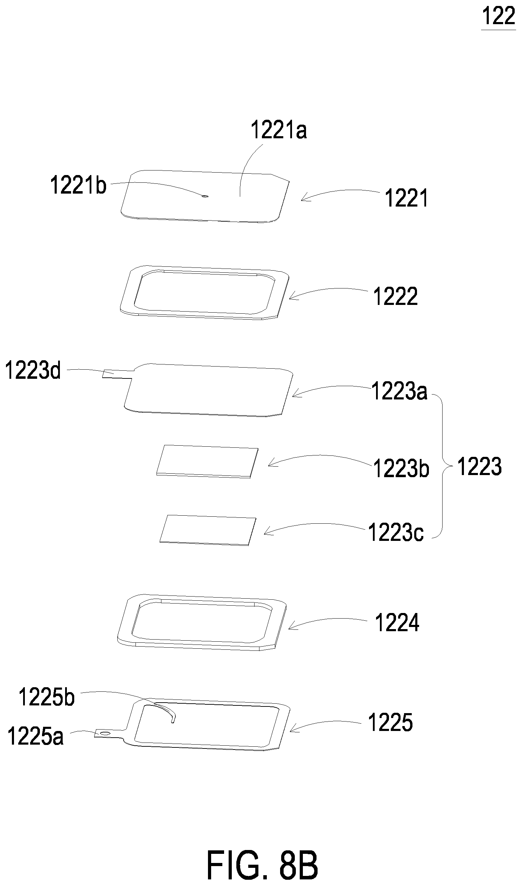

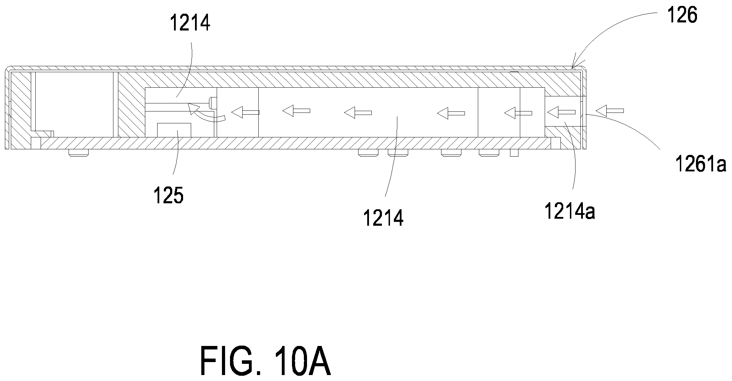

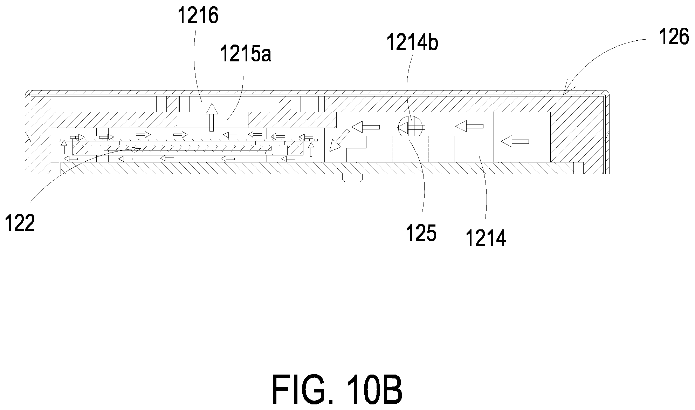

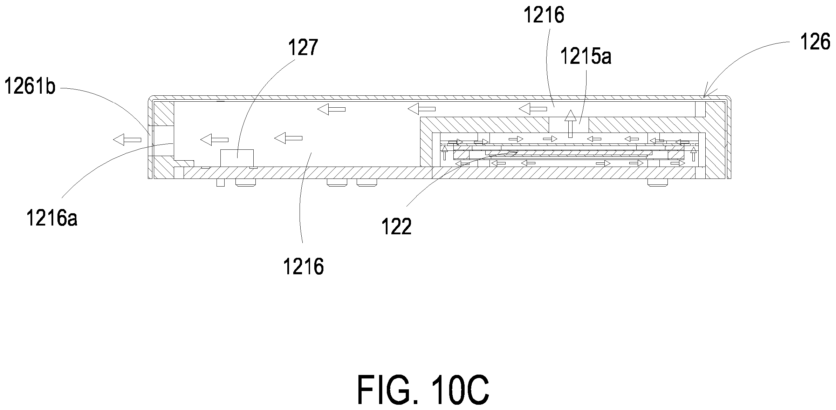

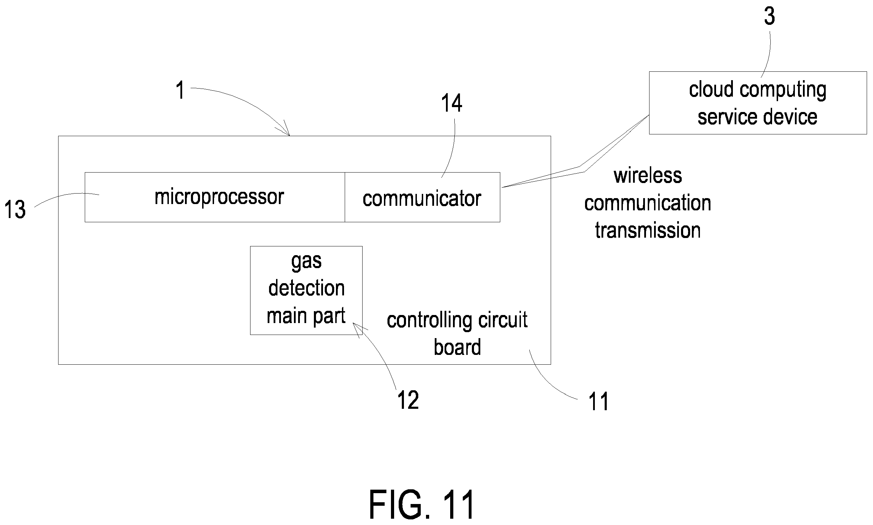

In the present disclosure, the specific implementation of the positive pressure control method of indoor air cleaning network mechanism system is understandable, and the structure of the gas detection module of the gas detector 1 of the present disclosure is described in detail below. Please refer to FIG. 3A to FIG. 11. In the embodiment, the gas detection module includes a controlling circuit board 11, a gas detection main part 12, a microprocessor 13 and a communicator 14. The gas detection main part 12, the microprocessor 13 and the communicator 14 are integrally packaged on the controlling circuit board 11 and electrically connected to each other. In the embodiment, the microprocessor 13 and the communicator 14 are mounted on the controlling circuit board 11. The microprocessor 13 controls the driving signal of the gas detection main part 12 for enabling the detection. In this way, the gas detection main part 12 detects the air pollution and outputs the air pollution information, and the microprocessor 13 receives, processes and provides the air pollution information to the communicator 14 for a communication transmission externally, and transmitting to the cloud computing server device 4 through IoT (Internet of Things) communication.

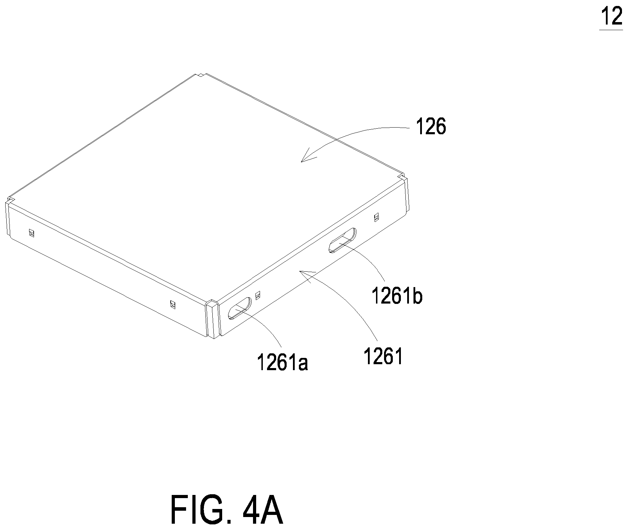

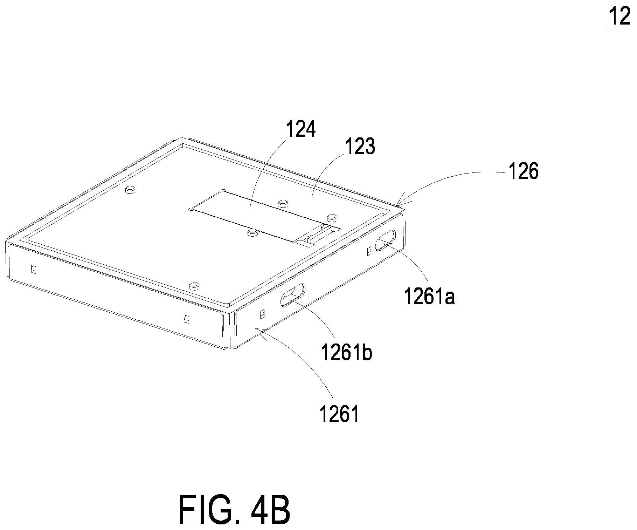

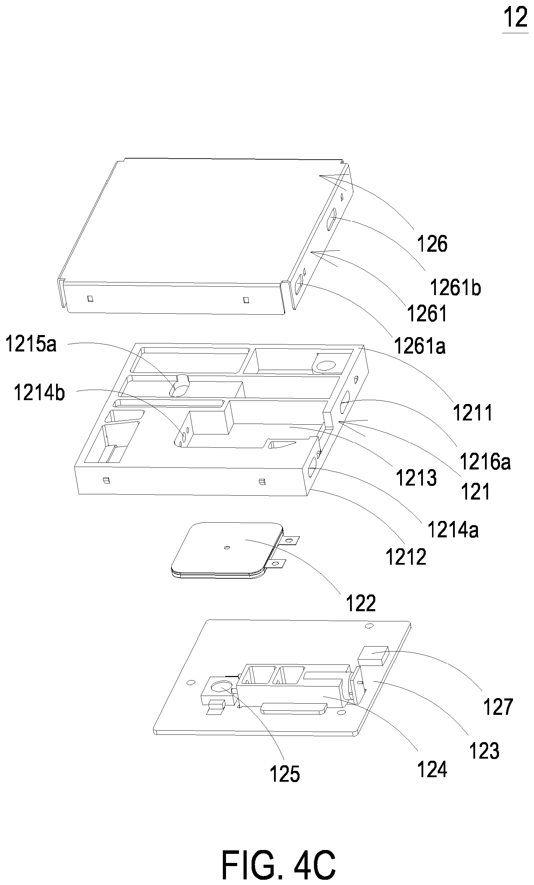

Please refer to FIG. 4A to FIG. 9A. In the embodiment, the gas detection main part 12 includes a base 121, a piezoelectric actuator 122, a driving circuit board 123, a laser component 124, a particulate sensor 125, and an outer cover 126. In the embodiment, the base 121 includes a first surface 1211, a second surface 1212, a laser loading region 1213, a gas-inlet groove 1214, a gas-guiding-component loading region 1215 and a gas-outlet groove 1216. The first surface 1211 and the second surface 1212 are two surfaces opposite to each other. In the embodiment, the laser loading region 1213 is hollowed out from the first surface 1211 toward the second surface 1212. The outer cover 126 covers the base 121 and includes a side plate 1261. The side plate 1261 has an inlet opening 1261a and an outlet opening 1261b. The gas-inlet groove 1214 is concavely formed from the second surface 1212 and disposed adjacent to the laser loading region 1213. The gas-inlet groove 1214 includes a gas-inlet 1214a and two lateral walls. The gas-inlet 1214a is in communication with an environment outside the base 121, and is spatially corresponding in position to an inlet opening 1261a of the outer cover 126. Two transparent windows 1214b are opened on the two lateral walls of the gas-inlet groove 1214 and are in communication with the laser loading region 1213. Therefore, the first surface 1211 of the base 121 is covered and attached by the outer cover 126, and the second surface 1212 is covered and attached by the driving circuit board 123, so that an inlet path is defined by the gas-inlet groove 1214. In the embodiment, the gas-guiding-component loading region 1215 mentioned above is concavely formed from the second surface 1212 and in communication with the gas-inlet groove 1214. A ventilation hole 1215a penetrates a bottom surface of the gas-guiding-component loading region 1215. The gas-guiding-component loading region 1215 includes four positioning protrusions 1215b disposed at four corners of the gas-guiding-component loading region 1215, respectively. In the embodiment, the gas-outlet groove 1216 includes a gas-outlet 1216a, and the gas-outlet 1216a is spatially corresponding to the outlet opening 1261b of the outer cover 126. The gas-outlet groove 1216 includes a first section 1216b and a second section 1216c. The first section 1216b is concavely formed out from the first surface 1211 in a region spatially corresponding to a vertical projection area of the gas-guiding-component loading region 1215. The second section 1216c is hollowed out from the first surface 1211 to the second surface 1212 in a region where the first surface 1211 is extended from the vertical projection area of the gas-guiding-component loading region 1215. The first section 1216b and the second section 1216c are connected to form a stepped structure. Moreover, the first section 1216b of the gas-outlet groove 1216 is in communication with the ventilation hole 1215a of the gas-guiding-component loading region 1215, and the second section 1216c of the gas-outlet groove 1216 is in communication with the gas-outlet 1216a. In that, when first surface 1211 of the base 121 is attached and covered by the outer cover 126 and the second surface 1212 of the base 121 is attached and covered by the driving circuit board 123, the gas-outlet groove 1216 and the driving circuit board 123 collaboratively define an outlet path.

In the embodiment, the laser component 124 and the particulate sensor 125 are disposed on and electrically connected to the driving circuit board 123 and located within the base 121. In order to clearly describe and illustrate the positions of the laser component 124 and the particulate sensor 125 in the base 121, the driving circuit board 123 is intentionally omitted. The laser component 124 is accommodated in the laser loading region 1213 of the base 121, and the particulate sensor 125 is accommodated in the gas-inlet groove 1214 of the base 121 and is aligned to the laser component 124. In addition, the laser component 124 is spatially corresponding to the transparent window 1214b, therefore, a light beam emitted by the laser component 124 passes through the transparent window 1214b and is irradiated into the gas-inlet groove 1214. A light beam path emitted from the laser component 124 passes through the transparent window 1214b and extends in an orthogonal direction perpendicular to the gas-inlet groove 1214. In the embodiment, a projecting light beam emitted from the laser component 124 passes through the transparent window 1214b and enters the gas-inlet groove 1214 to irradiate the suspended particles contained in the gas passing through the gas-inlet groove 1214. When the suspended particles contained in the gas are irradiated and generate scattered light spots, the scattered light spots are received and calculated by the particulate sensor 125 to obtain the gas detection information.

In the embodiment, the piezoelectric actuator 122 is accommodated in the square-shaped gas-guiding-component loading region 1215 of the base 121. In addition, the gas-guiding-component loading region 1215 of the base 121 is in fluid communication with the gas-inlet groove 1214. When the piezoelectric actuator 122 is enabled, the gas in the gas-inlet groove 1214 is inhaled by the piezoelectric actuator 122, so that the gas flows into the piezoelectric actuator 122, and is transported into the gas-outlet groove 1216 through the ventilation hole 1215a of the gas-guiding-component loading region 1215. Moreover, the driving circuit board 123 covers the second surface 1212 of the base 121, and the laser component 124 is disposed on the driving circuit board 123, and is electrically connected to the driving circuit board 123. The particulate sensor 125 is also disposed on the driving circuit board 123 and electrically connected to the driving circuit board 123. In that, when the outer cover 126 covers the base 121, the inlet opening 1261a is spatially corresponding to the gas-inlet 1214a of the base 121, and the outlet opening 126lb is spatially corresponding to the gas-outlet 1216a of the base 121.

In the embodiment, the piezoelectric actuator 122 includes a gas-injection plate 1221, a chamber frame 1222, an actuator element 1223, an insulation frame 1224 and a conductive frame 1225. In the embodiment, the gas-injection plate 1221 is made by a flexible material and includes a suspension plate 1221a and a hollow aperture 1221b. The suspension plate 1221a is a sheet structure and is permitted to undergo a bending deformation. Preferably but not exclusively, the shape and the size of the suspension plate 1221a are accommodated in the inner edge of the gas-guiding-component loading region 1215, but not limited thereto. The hollow aperture 1221b passes through a center of the suspension plate 1221a, so as to allow the gas to flow therethrough. Preferably but not exclusively, in the embodiment, the shape of the suspension plate 1221a is selected from the group consisting of a square, a circle, an ellipse, a triangle and a polygon, but not limited thereto.

In the embodiment, the chamber frame 1222 is carried and stacked on the gas-injection plate 1221. In addition, the shape of the chamber frame 1222 is corresponding to the gas-injection plate 1221. The actuator element 1223 is carried and stacked on the chamber frame 1222. A resonance chamber 1226 is collaboratively defined by the actuator element 1223, the chamber frame 1222 and the suspension plate 1221a and is formed between the actuator element 1223, the chamber frame 1222 and the suspension plate 1221a. The insulation frame 1224 is carried and stacked on the actuator element 1223 and the appearance of the insulation frame 1224 is similar to that of the chamber frame 1222. The conductive frame 1225 is carried and stacked on the insulation frame 1224, and the appearance of the conductive frame 1225 is similar to that of the insulation frame 1224. In addition, the conductive frame 1225 includes a conducting pin 1225a and a conducting electrode 1225b. The conducting pin 1225a is extended outwardly from an outer edge of the conductive frame 1225, and the conducting electrode 1225b is extended inwardly from an inner edge of the conductive frame 1225. Moreover, the actuator element 1223 further includes a piezoelectric carrying plate 1223a, an adjusting resonance plate 1223b and a piezoelectric plate 1223c. The piezoelectric carrying plate 1223a is carried and stacked on the chamber frame 1222. The adjusting resonance plate 1223b is carried and stacked on the piezoelectric carrying plate 1223a. The piezoelectric plate 1223c is carried and stacked on the adjusting resonance plate 1223b. The adjusting resonance plate 1223b and the piezoelectric plate 1223c are accommodated in the insulation frame 1224. The conducting electrode 1225b of the conductive frame 1225 is electrically connected to the piezoelectric plate 1223c. In the embodiment, the piezoelectric carrying plate 1223a and the adjusting resonance plate 1223b are made by a conductive material. The piezoelectric carrying plate 1223a includes a piezoelectric pin 1223d. The piezoelectric pin 1223d and the conducting pin 1225a are electrically connected to a driving circuit (not shown) of the driving circuit board 123, so as to receive a driving signal, such as a driving frequency and a driving voltage. Through this structure, a circuit is formed by the piezoelectric pin 1223d, the piezoelectric carrying plate 1223a, the adjusting resonance plate 1223b, the piezoelectric plate 1223c, the conducting electrode 1225b, the conductive frame 1225 and the conducting pin 1225a for transmitting the driving signal. Moreover, the insulation frame 1224 is insulated between the conductive frame 1225 and the actuator element 1223, so as to avoid the occurrence of a short circuit. Thereby, the driving signal is transmitted to the piezoelectric plate 1223c. After receiving the driving signal such as the driving frequency and the driving voltage, the piezoelectric plate 1223c deforms due to the piezoelectric effect, and the piezoelectric carrying plate 1223a and the adjusting resonance plate 1223b are further driven to generate the bending deformation in the reciprocating manner.

Furthermore, in the embodiment, the adjusting resonance plate 1223b is located between the piezoelectric plate 1223c and the piezoelectric carrying plate 1223a and served as a cushion between the piezoelectric plate 1223c and the piezoelectric carrying plate 1223a. Thereby, the vibration frequency of the piezoelectric carrying plate 1223a is adjustable. Basically, the thickness of the adjusting resonance plate 1223b is greater than the thickness of the piezoelectric carrying plate 1223a, and the vibration frequency of the actuator element 1223 can be adjusted by adjusting the thickness of the adjusting resonance plate 1223b.

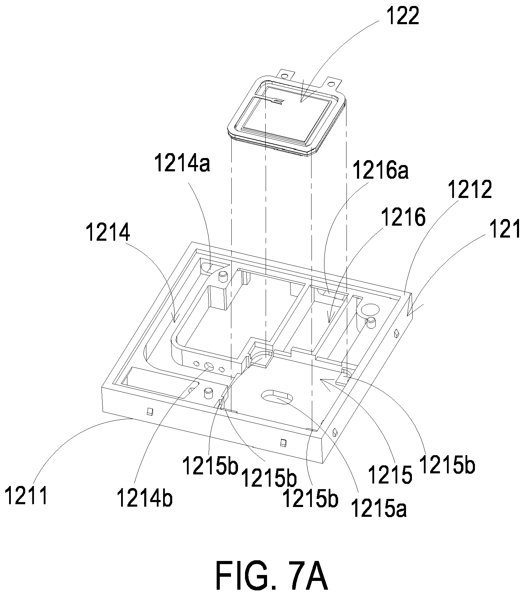

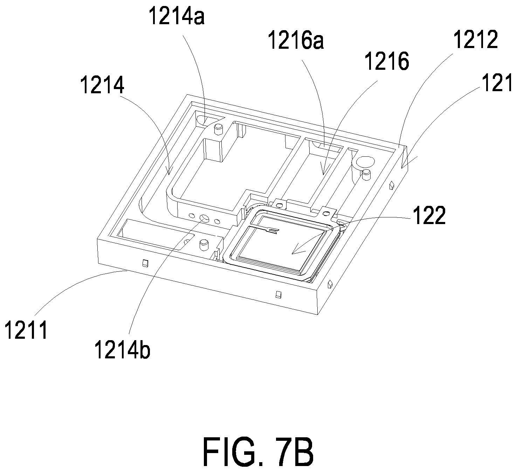

Please further refer to FIG. 7A, FIG. 7B, FIG.8A, FIG. 8B and FIG. 9A. In the embodiment, the gas-injection plate 1221, the chamber frame 1222, the actuator element 1223, the insulation frame 1224 and the conductive frame 1225 are stacked and positioned in the gas-guiding-component loading region 1215 sequentially, so that the piezoelectric actuator 122 is supported and positioned in the gas-guiding-component loading region 1215. A plurality of clearances 1221c are defined between the suspension plate 1221a of the gas-injection plate 1221 and an inner edge of the gas-guiding-component loading region 1215 for gas flowing therethrough. In the embodiment, a flowing chamber 1227 is formed between the gas-injection plate 1221 and the bottom surface of the gas-guiding-component loading region 1215. The flowing chamber 1227 is in communication with the resonance chamber 1226 between the actuator element 1223, the chamber frame 1222 and the suspension plate 1221a through the hollow aperture 1221b of the gas-injection plate 1221. By controlling the vibration frequency of the gas in the resonance chamber 1226 to be close to the vibration frequency of the suspension plate 1221a, the Helmholtz resonance effect is generated between the resonance chamber 1226 and the suspension plate 1221a, so as to improve the efficiency of gas transportation. When the piezoelectric plate 1223c is moved away from the bottom surface of the gas-guiding-component loading region 1215, the suspension plate 1221a of the gas-injection plate 1221 is driven to move away from the bottom surface of the gas-guiding-component loading region 1215 by the piezoelectric plate 1223c. In that, the volume of the flowing chamber 1227 is expanded rapidly, the internal pressure of the flowing chamber 1227 is decreased to form a negative pressure, and the gas outside the piezoelectric actuator 122 is inhaled through the clearances 1221c and enters the resonance chamber 1226 through the hollow aperture 1221b. Consequently, the pressure in the resonance chamber 1226 is increased to generate a pressure gradient. When the suspension plate 1221a of the gas-injection plate 1221 is driven by the piezoelectric plate 1223c to move toward the bottom surface of the gas-guiding-component loading region 1215, the gas in the resonance chamber 1226 is discharged out rapidly through the hollow aperture 1221b, and the gas in the flowing chamber 1227 is compressed, thereby the converged gas is quickly and massively ejected out of the flowing chamber 1227 under the condition close to an ideal gas state of the Benulli's law, and transported to the ventilation hole 1215a of the gas-guiding-component loading region 1215.

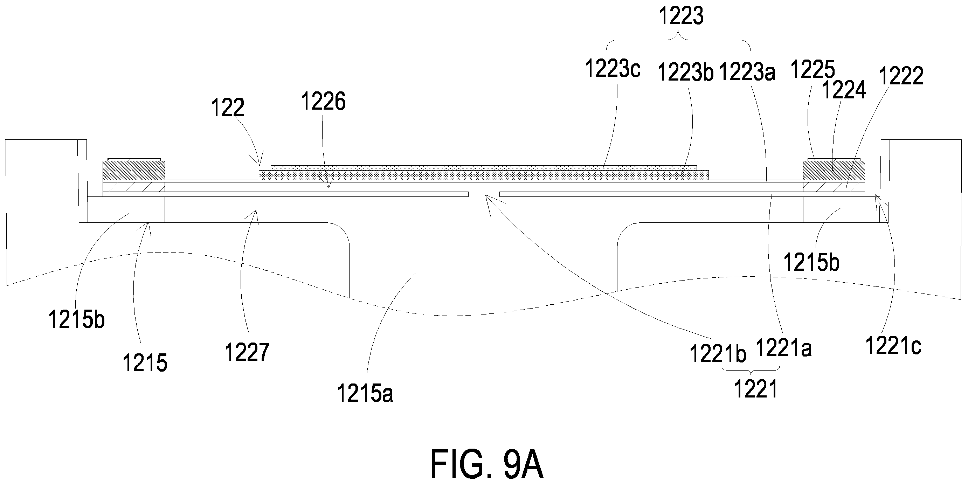

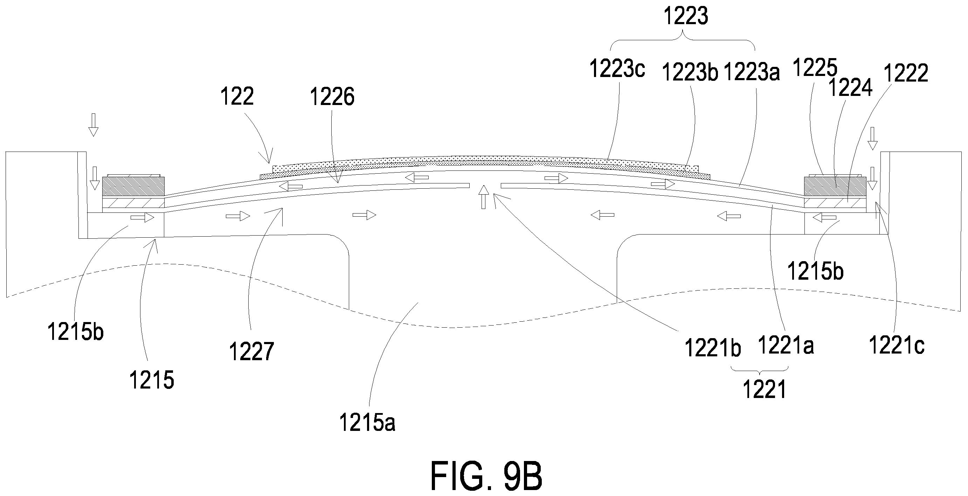

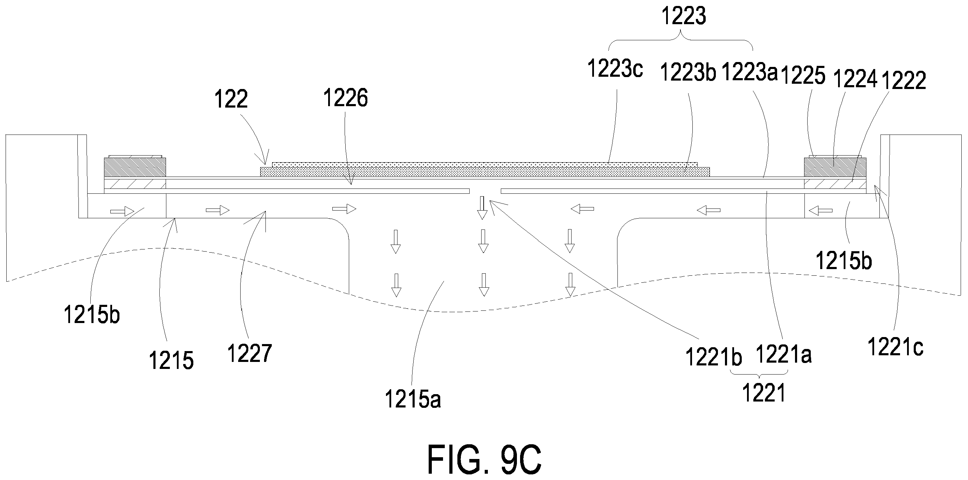

By repeating the above operation steps shown in FIG. 9B and FIG. 9C, the piezoelectric plate 1223c is driven to generate the bending deformation in a reciprocating manner. According to the principle of inertia, since the gas pressure inside the resonance chamber 1226 is lower than the equilibrium gas pressure after the converged gas is ejected out, the gas is introduced into the resonance chamber 1226 again. Moreover, the vibration frequency of the gas in the resonance chamber 1226 is controlled to be close to the vibration frequency of the piezoelectric plate 1223c, so as to generate the Helmholtz resonance effect to achieve the gas transportation at high speed and in large quantities. The gas is inhaled through the gas-inlet 1214a on the outer cover 126, flows into the gas-inlet groove 1214 of the base 121 through the gas-inlet 1214a, and is transported to the position of the particulate sensor 125. The piezoelectric actuator 122 is enabled continuously to inhale the gas into the inlet path, and facilitate the gas outside the gas detection module to be introduced rapidly, flow stably, and transported above the particulate sensor 125. At this time, a projecting light beam emitted from the laser component 124 passes through the transparent window 1214b to irritate the suspended particles contained in the gas flowing above the particulate sensor 125 in the gas-inlet groove 1214. When the suspended particles contained in the gas are irradiated and generate scattered light spots, the scattered light spots are received and calculated by the particulate sensor 125 for obtaining related information about the sizes and the concentration of the suspended particles contained in the gas. Moreover, the gas above the particulate sensor 125 is continuously driven and transported by the piezoelectric actuator 122, flows into the ventilation hole 1215a of the gas-guiding-component loading region 1215, and is transported to the gas-outlet groove 1216. At last, after the gas flows into the gas outlet groove 1216, the gas is continuously transported into the gas-outlet groove 1216 by the piezoelectric actuator 122, and thus the gas in the gas-outlet groove 1216 is pushed to discharge through the gas-outlet 1216a and the outlet opening 1261b.

The gas detector 1 of the present disclosure not only can detect the particulate matters in the gas, but also can detect the gas characteristics of the introduced gas, for example, to determine whether the gas is formaldehyde, ammonia, carbon monoxide, carbon dioxide, oxygen, ozone, or the like. Therefore, in one or some embodiments, the gas detector 1 of the present disclosure further includes a gas sensor 127 positioned and disposed on the driving circuit board 123, electrically connected to the driving circuit board 123, and accommodated in the gas-outlet groove 1216, so as to detect the air pollution introduced into the gas-outlet groove 1216. Preferably but not exclusively, in an embodiment, the gas sensor 127 includes a volatile-organic-compound sensor for detecting the information of carbon dioxide (CO₂) or volatile organic compounds (TVOC). Preferably but not exclusively, in an embodiment, the gas sensor 127 includes a formaldehyde sensor for detecting the information of formaldehyde (HCHO) gas. Preferably but not exclusively, in an embodiment, the gas sensor 127 includes a bacteria sensor for detecting the information of bacteria or fungi. Preferably but not exclusively, in an embodiment, the gas sensor 127 includes a virus sensor for detecting the information of virus in the gas. Preferably but not exclusively, the gas sensor 127 is a temperature and humidity sensor for detecting the temperature and humidity information of the gas.

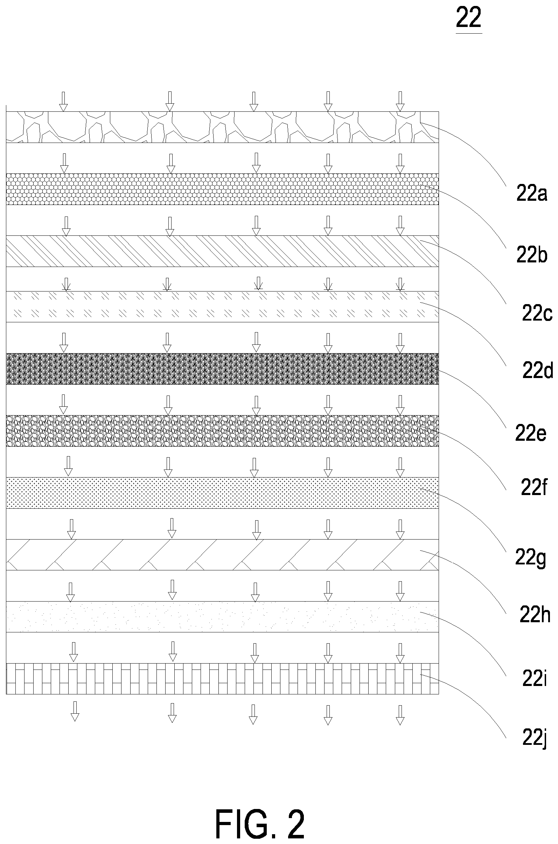

Please refer to FIG. 2. In the embodiment, the air guiding fan 21 of the indoor air cleaning network mechanism system is controlled to start and guide the air pollution to pass through the filter component 22 for filtration. Preferably but not exclusively, the filter component 22 is a filter with a grade of minimum filtration efficiency value (MREV) 8 or above, or a filter with high-efficiency particulate air (HEPA) grade, which is configured to absorb the chemical smoke, the bacteria, the dust particles and the pollen contained in the air pollution, so that the air pollution introduced into the filter component 22 is filtered and purified to achieve the effect of filtering and purification. Notably, in the present disclosure, the filter with the high efficiency particulate air (HEPA) grade is of 10 or above, and has a dust holding capacity greater than 12,000 mg. In an embodiment, the filter component 22 of the present disclosure is further combined with physical or chemical materials to provide a sterilization effect on the air pollution, and the airflow of the air guiding fan 21 flows in the path indicated by the arrow. In an embodiment, the filter component 22 includes a decomposition layer coated thereon to sterilize in chemical means. Preferably but not exclusively, the decomposition layer includes an activated carbon 22a configured to remove organic and inorganic substances in air pollution, and remove colored and odorous substances. Preferably but not exclusively, the activated carbon has a formaldehyde absorption capacity greater than 1,500 mg. In an embodiment, the decomposition layer includes a cleansing factor containing chlorine dioxide layer 22b configured to inhibit viruses, bacteria, fungi, influenza A, influenza B, enterovirus and norovirus in the air pollution, and the inhibition ratio can reach 99% and more, thereby reducing the cross-infection of viruses. In an embodiment, the decomposition layer includes an herbal protective layer 22c extracted from ginkgo and Japanese Rhus chinensis configured to resist allergy effectively and destroy a surface protein of influenza virus (such as H1N1 influenza virus) passing therethrough. In an embodiment, the decomposition layer includes a silver ion 22d configured to inhibit viruses, bacteria and fungi contained in the air pollution. In an embodiment, the decomposition layer includes a zeolite 23e configured to remove ammonia nitrogen, heavy metals, organic pollutants, Escherichia coli, phenol, chloroform and anionic surfactants. Furthermore, in some embodiments, the filter component 22 is combined with a light irradiation element to sterilize in chemical means. Preferably but not exclusively, the light irradiation element is a photo-catalyst unit including a photo catalyst 22f and an ultraviolet lamp 22g. When the photo catalyst 22f is irradiated by the ultraviolet lamp 22g, the light energy is converted into the chemical energy, thereby decomposes harmful gases and disinfects bacteria contained in the air pollution, so as to achieve the effects of filtering and purifying. Notably, the power of the ultraviolet lamp 22g in the present disclosure is more than 120 mw. In an embodiment, the light irradiation element is a photo-plasma unit including a nanometer irradiation tube 22h. When the introduced air pollution is irradiated by the nanometer irradiation tube 22h, the oxygen molecules and water molecules contained in the air pollution are decomposed into high oxidizing photo-plasma, and an ion flow capable of destroying organic molecules is generated. In that, volatile formaldehyde, volatile toluene and volatile organic compounds (VOC) contained in the air pollution are decomposed into water and carbon dioxide, so as to achieve the effects of filtering and purifying. Moreover, in some embodiments, the filter component 22 is combined with a decomposition unit to sterilize in chemical means. Preferably but not exclusively, the decomposition unit is a negative ion unit 22i with a dust collecting plate. It makes the suspended particles in the air pollution to carry with positive charge and adhered to the dust collecting plate carry with negative charges, so as to achieve the effects of filtering and purifying. Preferably but not exclusively, the decomposition unit is a plasma ion unit 22j. The oxygen molecules and water molecules contained in the air pollution are decomposed into positive hydrogen ions (H⁺) and negative oxygen ions (O²⁻) by the plasma ion. The substances attached with water around the ions are adhered on the surface of viruses and bacteria and converted into OH radicals with extremely strong oxidizing power, thereby removing hydrogen (H) from the protein on the surface of viruses and bacteria, and thus decomposing (oxidizing) the protein, so as to filter the introduced air pollution and achieve the effects of filtering and purifying.

In summary, the present disclosure provides a positive pressure control method of indoor air cleaning network mechanism system. A plurality of gas detectors are arranged in the indoor field and the outdoor field to detect air pollution information, carbon dioxide (CO₂) pressure detection information and temperature and humidity information of the air. At least one indoor air pollution processing device comprises at least one gas exchange device for implementing ventilation in the indoor field, at least one purification filter device for implementing complete clean room treatment, and at least one air conditioning device for implementing temperature and humidity adjustment in the indoor field. Each indoor air pollution processing device comprises at least one air guiding fan, at least one filter component and at least one driving controller. A networked cloud computing service device is provided to receive the air pollution information of the indoor field and the outdoor field, the carbon dioxide (CO₂) pressure detection information and the temperature and humidity information of the air through Internet of Things communication, and intelligently selecting and issuing a control instruction. Each indoor air pollution processing device with gas detectors is electrically connected to the networked cloud computing service device, so that an intelligent linkage system is formed. Real-time intelligent linkage control between the gas detectors in the indoor field and the outdoor field and the gas detector of the air pollution processing device is implemented to control actuation of the indoor air pollution processing device. In that, the air quality, the temperature and the humidity of space in the indoor felid are monitored anytime and anywhere, and the air pollution is guided to implement the purification and filtration. Meanwhile, the gas exchange device can obtain the result that whether the pressure difference of the carbon dioxide (CO₂) in the indoor field A and the outdoor field B reach the balance of zero pressure difference. Consequently, the positive pressure air intake is continuously provided in the indoor field to implement ventilation in indoor space, so that it prevents the air pollution in the outdoor field from entering the indoor field. When the balance of zero pressure difference is reached, the air guiding fan of the indoor air pollution processing device is controlled in real time to adjust the guide air volume, the energy-saving efficiency of the operation is effectively adjusted, and the air flow volume noise reaches zero specification value. Consequently, the instant detection of air pollution purification and the complete clean room treatment are realized, and the cleanliness of cleanroom grade is achieved. The present disclosure includes the industrial applicability and the inventive steps.

## Claims

1. A positive pressure control method of indoor air cleaning network mechanism system, **characterized by** comprising:
a) providing a plurality of gas detectors (1) arranged in an indoor field (A) and an outdoor field (B) to detect air pollution information, carbon dioxide (CO₂) pressure detection information and temperature and humidity information of the air;
b) providing at least one indoor air pollution processing device (2) arranged in the indoor field (A), and having at least one of the plurality of gas detectors (1) disposed therein, wherein each indoor air pollution processing device (2) comprises a air guiding fan (21), a filter component (22) and a driving controller (23), and the gas detector (1) is electrically connected to the driving controller (23), and receives the control instruction to the driving controller (23) through the Internet of Things communication to control actuation operation of the air guiding fan (21), wherein the indoor air pollution processing device (2) comprises at least one gas exchange device (2a) for implementing ventilation in the indoor field (A), at least one purification filter device (2b) for implementing complete clean room treatment, and at least one air conditioning device (2c) for implementing temperature and humidity adjustment in the indoor field (A);
c) providing a networked cloud computing service device (3) receiving the air pollution information of the indoor field (A) and the outdoor field (B), the carbon dioxide (CO₂) pressure detection information and the temperature and humidity information of the air through Internet of Things communication to store and form an air pollution big data database, and intelligently selecting and issuing a control instruction;
d) receiving the carbon dioxide (CO₂) pressure detection information and intelligently selecting to issue the control instruction by the networked cloud computing service device (3), wherein the carbon dioxide (CO₂) pressure detection information detected by the gas detectors (1) disposed inside the gas exchange device (2a), the gas detectors (1) arranged in the indoor field (A), and the gas detectors (1) arranged in the outdoor field (B) are transmitted to the networked cloud computing service device (3) through the Internet of Things communication, and intelligently calculates and compares the carbon dioxide (CO₂) pressure difference between the indoor field (A) and the outdoor field (B) according to the above carbon dioxide (CO₂) pressure detection information, so as to implement ventilation in indoor space (A);
e) comparing whether the pressure difference of the carbon dioxide (CO₂) in the indoor field (A) and the outdoor field (B) reach the balance of zero pressure difference, wherein if the balance of zero pressure difference is not reached, the control instruction is selectively issued to the gas detector (1) of the gas exchange device (2a), and the gas detector (1) of the gas exchange device (2a) receives the control instruction to control the actuation operation of the air guiding fan (21) through the driving controller (23), the positive pressure air intake is continuously provided in the indoor field (A) to implement ventilation in indoor space, so that it prevents the air pollution in the outdoor field (B) from entering the indoor field (A), when the balance of zero pressure difference is reached, the control instruction is issued to the gas detector (1) of the gas exchange device (2a), and the gas detector (1) of the gas exchange device (2a) receives the control instruction to stop the actuation operation of the air guiding fan (21) through the driving controller (23); and
f) receiving the control instruction and controlling the driving controller (23) to control the actuation operation of the air guiding fan (21) by the gas detectors (1) inside the purification filter device (2b) and the air conditioning device (2c), wherein the internal air pollution of the indoor field is continuously circulated and purified, so that the internal circulation air pollution purification and complete clean room treatment and the temperature and humidity adjustment of the indoor field (A) are implemented continuously, when the networked cloud computing service device (3) compares the pressure difference of carbon dioxide (CO₂) between the indoor field (A) and the outdoor field (B) to reach the balance of zero pressure difference, the networked cloud computing service device (3) issues the control instruction to the gas detectors (1) inside the purification filter device (2b) and the air conditioning device (2c) to control the air guiding fan (21) through the driving controller (23), wherein the air guiding fan (21) is controlled in real time to adjust the guide air volume, so that the instant detection of air pollution purification and the complete clean room treatment are realized, and the cleanliness of cleanroom grade is achieved.

2. The positive pressure control method of indoor air cleaning network mechanism system according to claim 1, wherein the gas exchange device (2a) is a fresh air fan, a full heat exchanger, or a heating, ventilation and air conditioning (HVAC), and the purification filter device (2b) comprises at least one purifier, at least one fan filter unit (FFU), at least one exhaust device, at least one extractor hood system, at least one dehumidifier, and at least one mobile vacuum cleaner.

3. The positive pressure control method of indoor air cleaning network mechanism system according to claim 1, wherein the air conditioning device (2c) sets the temperature and humidity at safety values to maintain the temperature at 25°C±3°C and the humidity at 50%±10%.

4. The positive pressure control method of indoor air cleaning network mechanism system according to claim 1, wherein Internet of Things communication is a wireless communication for communicating with the networked cloud computing service device (3) via a wireless connection, the wireless communication transmission comprises one selected from the group consisting of a Wi-Fi module, a Bluetooth module, a radio frequency identification module and a near field communication (NFC) module.

5. The positive pressure control method of indoor air cleaning network mechanism system according to claim 1, wherein Internet of Things communication is a wired communication for communicating with the networked cloud computing service device (3) via a wired line.

6. The positive pressure control method of indoor air cleaning network mechanism system according to claim 1, wherein the gas detector (1) comprises a controlling circuit board (11), a gas detection main part (12), a microprocessor (13) and a communicator (14), the controlling circuit board (11) is electrically connected to the driving controller (23), and the gas detection main part (12), the microprocessor (13) and the communicator (14) are integrally packaged on the controlling circuit board (11) and electrically connected to the controlling circuit board (11), wherein the microprocessor (13) controls the detection of the gas detection main part (12), the gas detection main part (12) detects the air pollution, and the microprocessor (13) processes the air pollution to output and provide the air pollution information to the communicator (14) for a communication transmission externally.

7. The positive pressure control method of indoor air cleaning network mechanism system according to claim 1, wherein the filter component (22) is a filter with a minimum efficiency reporting value (MREV) of 8 or above.

8. The positive pressure control method of indoor air cleaning network mechanism system according to claim 1, wherein the filter component (22) is a filter with a high efficiency particulate air (HEPA) grade, wherein the filter with the high efficiency particulate air (HEPA) grade is of 10 or above, and has a dust holding capacity greater than 12,000 mg.

9. The positive pressure control method of indoor air cleaning network mechanism system according to claim 1, wherein the filter component (22) is combined with a decomposition layer a decomposition layer coated thereon, and the decomposition layer uses chemical means to sterilize the air pollution, and the decomposition layer is composed of an activated carbon or a cleansing factor containing chlorine dioxide layer, wherein the activated carbon has a formaldehyde absorption capacity greater than 1,500 mg.

10. The positive pressure control method of indoor air cleaning network mechanism system according to claim 1, wherein the filter component (22) is combined with a light irradiation element to sterilize in chemical means, and the light irradiation element is a photo-catalyst unit including a photo catalyst and an ultraviolet lamp, wherein the power of the ultraviolet lamp is more than 120 mw.

11. The positive pressure control method of indoor air cleaning network mechanism system according to claim 1, wherein the networked cloud computing service device (3) further comprises an artificial intelligence generated content (AIGC) model (35), the artificial intelligence generated content (AIGC) model (35) provides professional generated data and user generated data to the indoor air cleaning network mechanism system, wherein the professional generated data includes outdoor and indoor air pollution standard data of the buildings, indoor field space data of the buildings, clean room grade standard data, and required software and hardware specifications, the user generated data includes indoor field air pollution data of a user's building, indoor field experimental measurement air pollution data of the user's building, and air exchange rate data of heating, ventilation and air conditioning (HVAC) in the user's building, wherein the artificial intelligence generated content (AIGC) mode (35) calculates, compares and identifies the professional generated data and the user generated data to form automatically-generated data, and the automatically-generated data includes the optimized number of gas purification device hardware, the optimized performance control of gas purification device hardware, the optimized noise reduction control of gas purification device hardware, the minimized one-time installation cost of the air cleaning system and the minimized operation cost of the air cleaning system.

12. The positive pressure control method of indoor air cleaning network mechanism system according to claim 1, wherein the clean room level is ZAPClean Room 1~12.
